# EUROPÄISCHE PATENTSCHRIFT

(11) **EP 0 906 324 B1**
(45) Veröffentlichungstag und Bekanntmachung des Hinweises auf die Patenterteilung: **01.08.2001**
(21) Anmeldenummer: 97923046.3
(22) Anmeldetag: 09.05.1997
(51) Int. Cl.: C07H 5/10, C07H 3/06, A61K 31/70, C08B 37/00

(54) **KOHLENHYDRATDERIVATE UND IHRE SYNTHESE AN FESTER PHASE**
CARBOHYDRATE DERIVATIVES AND THEIR SOLID-PHASE SYNTHESIS
DERIVES GLUCIDIQUES ET LEUR SYNTHESE EN PHASE SOLIDE

(30) Priorität: 24.05.1996 DE 19621177
(43) Veröffentlichungstag der Anmeldung: 07.04.1999
(73) Patentinhaber: BASF AKTIENGESELLSCHAFT, 67056 Ludwigshafen (DE)
(72) Erfinder: SCHMIDT, Richard, R., D-78465 Konstanz (DE); RADEMANN, Jörg, CH-8280 Kreuzlingen (CH)
(86) Internationale Anmeldenummer: EP9702393
(87) Internationale Veröffentlichungsnummer: WO9745436

(56) Entgegenhaltungen:
- WO-A-95/03315
- US-A- 5 237 016
- US-A- 5 399 501
- US-A- 5 412 087
- M.SCHUSTER ET AL.: "Solid-Phase Chemical-Enzymatic Sythesis of Glycopeptides and Oligosaccharides." JOURNAL OF THE AMERICAN CHEMICAL SOCIETY., Bd. 116, Nr. 3, 9.Februar 1994, DC US, Seiten 1135-1136, XP002044053 in der Anmeldung erwähnt
- L.YAN ET AL.: "Glycosylation on the Merrifield Resin Using Anomeric Sulfoxides." JOURNAL OF THE AMERICAN CHEMICAL SOCIETY., Bd. 116, Nr. 15, 27.Juli 1994, DC US, Seiten 6953-6954, XP002044054 in der Anmeldung erwähnt
- CHEMICAL ABSTRACTS, vol. 91, no. 5, 1979 Columbus, Ohio, US; abstract no. 35296g, R.T.LEE ET AL.: "A Simple Method for the Preparation of Polyacrylamide Gels Containing Thioglycoside Ligands." Seite 279; Spalte 2; XP002044055 & ANALYTICAL BIOCHEMISTRY, Bd. 95, Nr. 1, 1979, Seiten 260-269,

## Beschreibung

Die Erfindung betrifft Kohlenhydratderivate, ein Verfahren zu ihrer Herstellung und ihre Verwendung.

In der klassischen Wirkstoffsuchforschung wurde die biologische Wirkung neuer Verbindungen in einem Zufalls-Screening am ganzen Organismus beispielsweise der Pflanze oder dem Mikroorganismus getestet. Dabei war die biologische Testung gegenüber der Synthesechemie der limitierende Faktor. Durch die Bereitstellung molekularer Testsysteme durch die Molekular- und Zellbiologie hat sich die Situation drastisch verändert.

Für die moderne Wirkstoffsuchforschung wurden und werden zur Zeit eine Vielzahl von molekularen Testsystemen wie beispielsweise Rezeptorbindungsassays, Enzymassays und Zell-Zellinteraktionsassays entwickelt. Die Automatisierung und Miniaturisierung dieser Testsysteme ermöglicht einen hohen Probendurchsatz. Durch diese Entwicklung läßt sich in immer kürzerer Zeit eine immer größere Anzahl an Chemikalien auf ihre biologische Wirkung im Zufalls-Screening und damit auf eine mögliche Verwendung als Leitstruktur für einen Wirkstoff in der Medizin, Tiermedizin oder im Pflanzenschutz testen.

Ein modernes automatisiertes Testsystem ermöglicht in einem Massenscreening die Prüfung von 100.000 und mehr Chemikalien pro Jahr auf ihre biologische Wirkung.

Die klassische Synthesechemie wurde durch diese Entwicklung zum limitierenden Faktor in der Wirkstoffsuchforschung.

Soll die Leistungsfähigkeit dieser Testsysteme voll ausgeschöpft werden, muß die Effizienz der chemischen Wirkstoffleitstruktursynthese beträchtlich gesteigert werden.

Zu dieser erforderlichen Effizienzsteigerung kann die kombinatorische Chemie einen Beitrag leisten, insbesondere wenn sie sich automatisierter Festphasensynthesemethoden bedient (s. z.B. Übersichtsartikel J. Med. Chem. 1994, 37, 1233 und 1994, 37, 1385). Die kombinatorische Chemie ermöglicht die Synthese einer breiten Vielfalt unterschiedlicher chemischer Verbindungen, sogenannter Substanzbibliotheken. Die Synthese an der Festphase hat den Vorteil, daß Nebenprodukte und überschüssige Reaktanten leicht entfernt werden können, so daß keine aufwendige Reinigung der Produkte notwendig ist. Die fertigen Syntheseprodukte können direkt, d.h. trägergebunden, oder nach Abspaltung von der festen Phase dem Massenscreening zugeführt werden. Auch Zwischenprodukte können im Massenscreening geprüft werden.

Bisher beschriebene Anwendungen beschränken sich überwiegend auf das Peptid- und Nukleotidgebiet (Lebl et al., Int. J. Pept. Prot. Res. 41, 1993: 203 und WO 92/00091) oder ihre Derivate (WO 96/00391). Da Peptide und Nukleotide als Pharmaka wegen ihrer ungünstigen pharmakologischen Eigenschaften nur begrenzt einsetzbar sind, ist es wünschenswert, die von der Peptid- und Nukleotidchemie her bekannten und bewährten Festphasensynthesemethoden für die synthetische organische Chemie zu nutzen.

In US 5 288 514 wird über eine der ersten kombinatorischen Festphasensynthesen in der organischen Chemie außerhalb der Peptid-und Nukleotidchemie berichtet. US 5 288 514 beschreibt die sequentielle Synthese von 1,4-Benzodiazepinen an fester Phase.

WO 95/16712, WO 95/30642 und WO 96/00148 beschreiben weitere Festphasensynthesen potentieller Wirkstoffe in der kombinatorischen Chemie.

Um die Möglichkeiten der modernen Testsysteme im Massenscreening voll ausnutzen zu können, ist es jedoch notwendig, ständig neue Verbindungen mit einer möglichst hohen strukturellen Diversität in das Massenscreening einzuspeisen. Durch dieses Vorgehen läßt sich die Zeit zur Identifizierung und Optimierung einer neuen Wirkstoffleitstruktur beträchtlich verkürzen.

Es ist deshalb erforderlich, ständig neue diverse, kombinatorische Festphasensynthesen zu entwickeln. Dabei ist es sinnvoll, sich an biologisch wirksamen Verbindungen zu orientieren.

Kohlenhydrate und deren Derivate sind in vielen Bereichen gesuchte, schwer zu synthetisierende Verbindungen. So werden einige Polysaccharide wie beispielsweise das Schizophylhan als Antitumormittel verwendet.

Eine Vielzahl von Antibiotika besitzen Kohlenhydratreste wie Antibiotika aus der Gruppe der Makrolide, Anthracycline oder Endiine oder bestehen vollständig aus Kohlenhydraten wie das Streptomycin, das z.B. in der Tiermedizin oder bei der Behandlung von Pflanzenkrankheiten verwendet wird.

Glycokonjugate wie Glycoproteine und/oder Glycolipide spielen eine entscheidende Rolle bei der Zell-Zell-Interaktion, bei der Transformation von normalen Körperzellen zu Tumorzellen und bei entzündlichen oder allergenen Prozessen im Körper. So ist beispielsweise Sialyl-Lewis X ein intensiv beforschtes Glycokonjugat, das sich durch seine entzündungshemmende Wirkung auszeichnet.

Von Schuster et al. (Abstr. Pap. Am. Chem. Soc., 1994, 207 Meet. Pt. 1, CARB29 und J. Am. Chem. Soc., 1994, 116, 1135-36) wird eine enzymatische Festphasensynthese eines Kohlenhydratderivats mittels Glycosyltransferasen beschrieben. Schuster et al. benutzen für ihre Synthese ein Glycopeptid, das über einen durch Proteasen spaltbaren Linker an einen Aminopropylsilicagelträger gebunden wurde. Dieses Glycopeptid dient als Akzeptor für Glycosyltransferasen katalysierte Glycosylierungsreaktionen zum Aufbau eines Sialyl-Lewis X-Restes. Von Nachteil bei dieser Methode ist, daß Glycosyltransferasen empfindliche, nicht allgemein und in beliebiger Menge zugängliche enzymatische Katalysatoren sind. Ein vollständiger Umsatz der Reaktanten konnte nicht erreicht werden, so daß Reaktionsgemische nach der Abspaltung erhalten wurden. Ihre hohe, für die einzelne Reaktion vorteilhafte Spezifität führt dazu, daß nicht jeder Zucker und jede Position an den verschiedenen Zuckern durch enzymatische Glycosylierungsreaktionen zugänglich ist. Der verwendete Protease spaltbare Linker ist für die chemische Zuckersynthese nur bedingt geeignet, so daß eine Kombination von chemischer und enzymatischer Zuckersynthese um alle Zucker und Positionen am Zucker gleich gut zu erreichen, mit diesem Linker nicht möglich ist.

In J. Am. Chem. Soc. (1994, 116, 6953 ff.) beschreibt Kahn et al. eine Kohlenhydratsynthese an einem Thiophenolharz. Nachteilig bei dieser Methode ist, daß die Abspaltung des Glycosids die Verwendung des giftigen und schwer abtrennbaren Quecksilbertrifluoracetats erfordert. Dadurch ist eine weitere Aufreinigung der Syntheseprodukte erforderlich. Die Abspaltung des Glycosids vom Träger führt außerdem nur zu den freien Zuckern, ohne daß an der gespaltenen Bindung eine neue Bindung geknüpft und damit ein neuer Substituent eingeführt werden kann.

Die Glycosylierungsreaktion läßt sich bei dieser Methode nur mit hochreaktiven Sulfoxiden bei tiefen Temperaturen durchführen, wobei außerdem sehr stabile Schutzgruppen erforderlich sind.

Die von Daniskefsky et al. beschriebene Festphasensynthese (Science. 260, 1993, 1307 ff und 269, 1995, 202 ff) von Glycosiden sind auf Glycale als Edukte d.h. als Donoren beschränkt. Außerdem lassen sich nur 1-6 glycosidische Bindungen knüpfen und wie bei Kahn et al. kann bei der Abspaltung vom Träger kein neuer weiterer Substituent eingeführt werden.

Aufgabe der vorliegenden Erfindung war es, ein schnelles und effizientes Herstellverfahren von Kohlenhydratderivaten an der festen Phase bereitzustellen, das die oben genannten Nachteile nicht aufweist und die Anforderungen der kombinatorischen Chemie erfüllt.

Es wurde nun ein Verfahren zur Herstellung von Kohlenhydratderivaten der Formel I gefunden 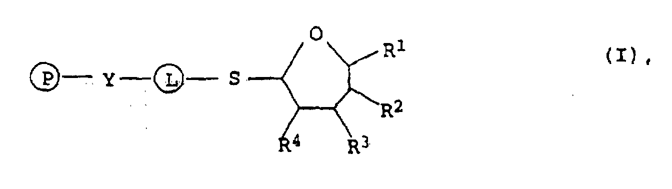 in der die Variable und Substituenten folgende Bedeutung haben:
- (P): eine feste Phase
- (L): ein aliphatischer Linker mit 2 bis 12 Kohlenstoffatomen,
- R¹: CHR⁵R⁶, R⁵
- R², R³, R⁴: unabhängig voneinander Wasserstoff, XH, substituiertes oder unsubstituiertes 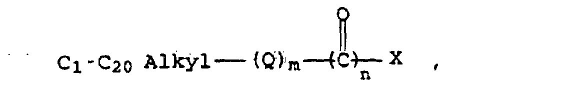 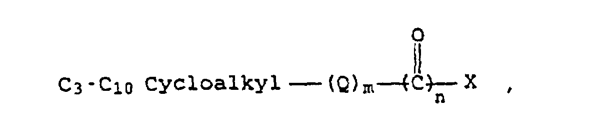 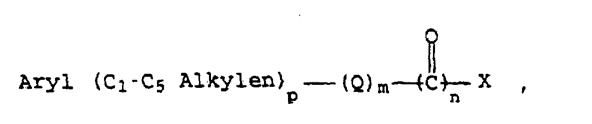 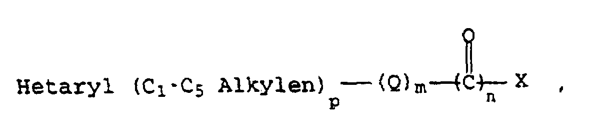 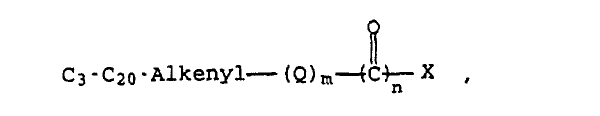 (C₁-C₄-Alkyl)₃SiO, Diaryl (C₁-C₅-Alkyl) SiO, Aryl(C₁-C₅-Dialkyl)SiO oder R⁷ und m = 0,1; n = 0,1; p = 0,1
oder zwei benachbarte Reste R², R³, R⁴, R⁶ unabhängig voneinander ein substituiertes oder unsubstituiertes Arylalkylidenacetal oder ein Alkylidenacetal bilden,
- R⁵: Wasserstoff, substituiertes oder unsubstituiertes C₁-C₂₀-Alkyl, C₃-C₂₀ Alkenyl, Arylalkylen oder Aryl,
- R⁶: Wasserstoff, XH, substituiertes oder unsubstituiertes 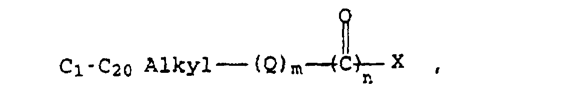 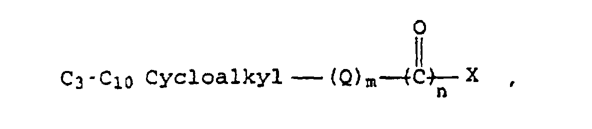 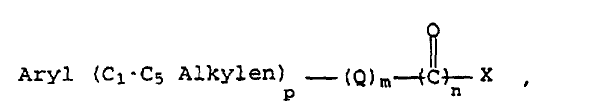 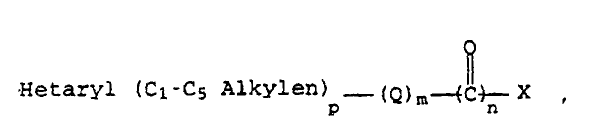 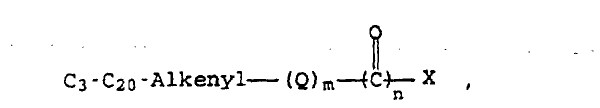 (C₁-C₄-Alkyl)₃SiO, Diaryl(C₁-C₅-Alkyl)SiO, Aryl(C₁-C₅-Dialkyl)SiO oder R⁷ und m = 0,1; n = 0,1; p = 0,1 oder (Aryl)₃CO,
- R⁷: eine Verbindung der Formel IV bedeutet 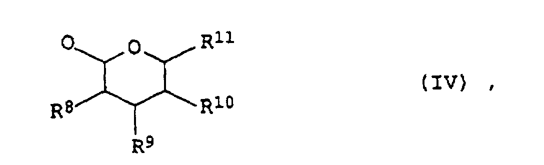 wobei die Substituenten R⁸, R⁹, R¹⁰, R¹¹ folgende Bedeutung haben:
R⁸, R⁹, R¹⁰ unabhängig voneinander, die für die Reste R², R³, R⁴ beschriebene Bedeutung haben und gleich oder verschieden von den Resten R², R³, R⁴ sein können,
R¹¹ CHR⁵R⁶, R⁵
   oder zwei benachbarte Reste R⁸, R⁹, R¹⁰, R⁶ unabhängig voneinander ein substituiertes oder unsubstituiertes Arylalkylidenacetal oder ein Alkylidenacetal bilden können,
- Q: O, NH
- X: O, NR¹²
- Y: O, S
- R¹²: Wasserstoff, substituiertes oder unsubstituiertes C₁-C₁₀Alkyl, C₃-C₈-Cycloalkyl, Aryl, Hetaryl, Arylalkyl
dadurch gekennzeichnet, daß man Verbindungen der Formel II 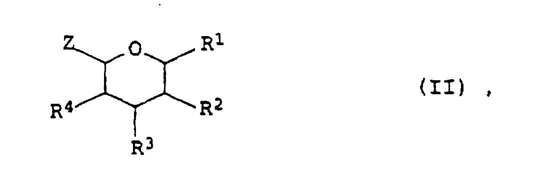 in der Z ausgewählt ist aus der Gruppe
N³, Halogen, 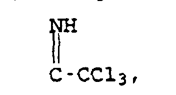 OCH₂CH₂CH₂CH=CH₂, OPO₂H₂, S-Aryl, S-Alkyl
bedeutet, in Gegenwart eines Promotors an eine funktionalisierte feste Phase der Formel III 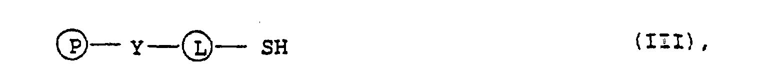 koppelt.

Die Erfindung betrifft außerdem neue Kohlenhydratderivate und ihre Verwendung.

Als feste Phase (P) können in dem erfindungsgemäßen Verfahren Träger, wie sie aus der Festphasen-Peptidsynthese bekannt sind, verwendet werden. Nutzbare Träger können, soweit sie mit der verwendeten Synthesechemie kompatibel sind aus einer Vielzahl von Materialien bestehen. Die Größe der Träger kann je nach Material in weitem Rahmen variiert werden. Bevorzugt werden Partikel im Bereich von 1 µm bis 1,5 cm als Träger verwendet, besonders bevorzugt bei polymeren Trägern Partikel im Bereich zwischen 1 µm und 150 µm.

Die Form der Träger ist beliebig, bevorzugt sind sphärische Partikel. Die Träger können in ihrer Größenverteilung homogen oder heterogen sein, bevorzugt sind homogene Partikelgrößen.

Geeignete feste Phasen (P) sind beispielsweise Keramik, Glas, Latex, funktionalisierte quervernetzte Polystyrole, Polyacrylamide, Silicagele, oder Harze.

Um eine Anknüpfung des Reaktanten bzw. eine Abspaltung des Syntheseproduktes nach der Synthese zu ermöglichen, muß der Träger geeignet funktionalisiert oder mit einem Linker versehen sein, der eine entsprechende funktionelle Gruppe besitzt. Bevorzugt geeignete Träger bzw. Träger-Linker-Konjugate sind beispielsweise Chlorbenzylharz (Merrifieldharz), Rink-Harz (Novabiochem), Sieber-Harz (Novabiochem), Wang-Harz (Bachem), Tentagel-Harze (Rapp-Polymere), Pega-Harz (Polymer Laboratories) oder Polyacrylamide. Besonders bevorzugt ist als Träger Chlorbenzylharz.

Zur Anknüpfung des bevorzugten aliphatischen Linkers (Formel VI) 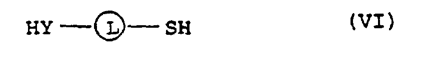 mit 2 bis 12 Kohlenstoffatomen, wobei Y Sauerstoff oder Schwefel bedeutet, an die feste Phase, ist diese gegebenenfalls in dem Fachmann bekannterweise zu modifizieren.

Beispielsweise können feste Phasen auf Basis von Polyacrylamiden mit 4-Chlormethylbenzoesäure so derivatisiert werden, daß der Linker (= Formel VI) an die feste Phase angeknüpft werden kann und diese so in geeigneter Weise für die Synthese funktionalisiert werden kann (Schema I).

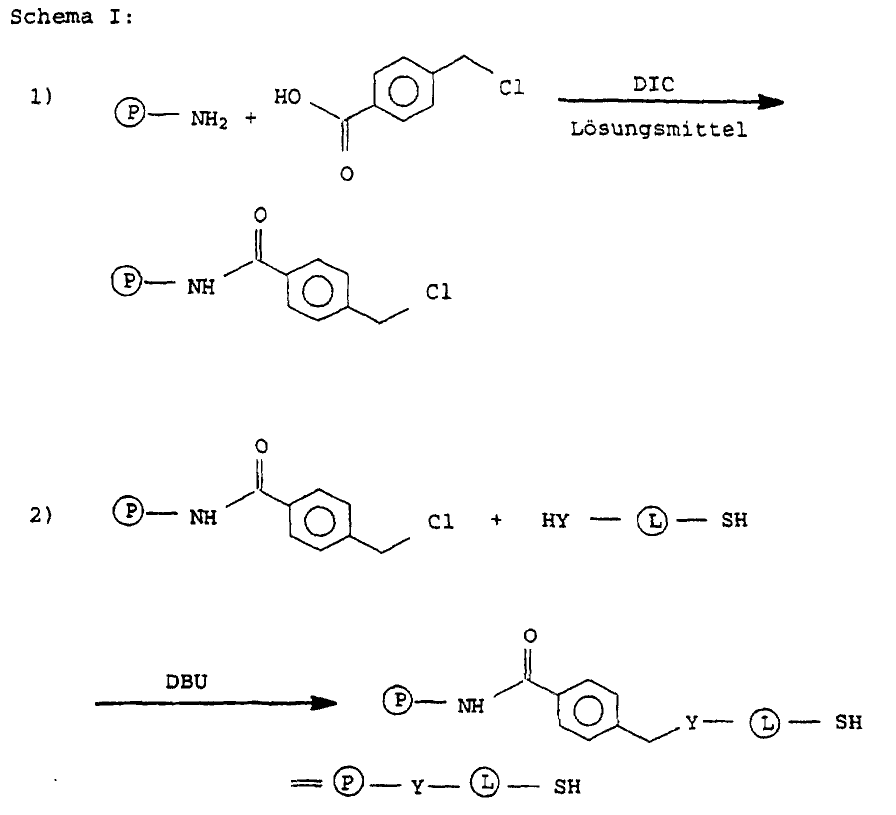

Die Amidbindung (1) zwischen dem Träger und der 4-Chlormethylbenzoesäure kann in Lösungsmittel mit Hilfe von beispielsweise Diisopropylcarbodiimid (=DIC) geknüpft werden. Weitere zur Knüpfung der Amidbindung geeigneten Kupplungsreagenzien sind beispielsweise TBTU, HBTU, BOP oder PYBOP (Lit.: Int. J. Peptide Prot. Rev. 35. 1990: 161-214). Ist Y ein Sauerstoff, wird der vorhandene Schwefel des Linkers mit einer Schutzgruppe z. B. Trityl versehen. Diese Technik kann auch im Falle von Y gleich Schwefel verwendet werden. Die Kupplung des bevorzugten Linkers mit Y = S an den Träger kann aber auch direkt ohne Schutzgruppe erfolgen.

Als Lösungsmittel für die Bildung der funktionalisierten festen Phase eignen sich aprotische, unpolare oder polare Lösungsmittel, beispielsweise DMF, CH₂Cl₂, DMSO oder THF. Es können einzelne Lösungsmittel oder Gemische verwendet werden.

Zur Bestimmung der Konzentration der Thiolgruppen der Linker verknüpften festen Phase wurde durch Elementaranalyse der Prozentgehalt der verschiedenen Elemente wie C, H, S usw. bestimmt. Der S-Gehalt der festen Phase ist ein Maß für die freien Thiolgruppen am Träger. Die Thiolgruppenkonzentration liegt im Bereich von 0,05 bis 0,9 mMol/g Harz, bevorzugt zwischen 0,2 bis 0,6 mMol/g Harz.

Der Linker (= L) kann verzweigt oder unverzweigt, chiral oder achiral sein.

Als Dithiole seien beispielsweise 1,3-Propandithiol, 1,2-Propandithiol, 1,2-Butandithiol, 1,3-Butandithiol, 1,4-Butandithiol, 2,3-Butandithiol, 1,2-Pentandithiol, 1,3-Pentandithiol, 1,4-Pentandithiol, 1,5-Pentandithiol, 2,4-Pentandithiol, 2-Methyl-1,4-Butandithiol, 1,2-Hexandithiol, 1,3-Hexandithiol, 1,4-Hexandithiol, 1,5-Hexandithiol, 1,6-Hexandithiol, 2,3-Hexandithiol, 2,4-Hexandithiol, 2,5-Hexandithiol, 2-Methyl-1,5-Pentandithiol, 1,5-Dithiomethylcyclohexan oder 3-Methyl-1,5-Pentandithiol genannt.

Die Reaktion (2) wird zwischen 30 und 100°C, bevorzugt zwischen 20 und 50°C durchgeführt.

Die Abspaltung des Syntheseprodukts I von der festen funktionalisierten Phase kann in Gegenwart mindestens eines thiophilen Reagenz und mindestens eines Alkohols erfolgen.

Als thiophile Reagentien kommen beispielsweise Dimethyl-(methylthio-)sulfoniumtriflat (= DMTST) oder das korrespondierende Dimethyl-(methylthio-)tetrafluorborat (=DMTSB) in Frage. Weitere geeignete Reagentien werden z.B. von Waldmann H. in Nachr. Chem. Tech. Lab. 39, 1991, 675-682 beschrieben. Das Syntheseprodukt I läßt sich außerdem leicht vom Linker der funktionalisierten festen Phase mit Bromonium-Ionen in Lösungsmittel : Wassergemischen abspalten, bevorzugt Aceton : Wasser besonders bevorzugt Aceton : Wasser (9:1), dabei werden die freien Zucker erhalten. Als Alkohole kommen prinzipiell alle Alkohole wie primäre oder sekundäre Alkohole, ein- oder mehrwertige Alkohole in Frage. Beispielhaft seien hier verzweigte oder unverzweigte, gesättigte oder einfach oder mehrfach ungesättigte C₁-C₂₀-Alkohole wie Methanol, Ethanol, 1-Propanol, 2-Propanol, 1-Butanol. 2-Butanol, 2-Methyl-1-Propanol, 1-Pentanol, 2-Pentanol, 3-Pentanol, 2-Methyl-1-Butanol, 3-Methyl-1-Butanol. 2-Methyl-3-Butanol, 3-Methyl-2-Butanol, Hexanol, Heptanol, Oktanol, Nonanol, Dekanol, Undekanol, Dodekanol, Tridekanol, Tetradekanol, Pentadekanol, Hexadekanol, Heptadekanol, Oktadekanol, Nonadekanol, Eicosanol oder von Fettsäuren wie Palmitinsäure, Palmitoleinsäure, Stearinsäure, Ölsäure, Eicosatriensäure, Linolsäure, Linolensäure, Arachidonsäure abgeleitete Fettalkohole genannt. Weiter seien beispielhaft Alkohole aus dem Terpenoidstoffwechsel genannt wie Menthol, Geraniol, Farnesol oder mehrwertige Alkohole wie Glycol, Glycerin, Pentite wie Adonit, Arabit oder Xylit, Hexite wie Sorbit, Dulcit oder Mannit.

Ebenfalls geeignet sind Hydroxycarbonsäuren wie Milchsäure, Mandelsäure, Pantoinsäure, 3-Phenylmilchsäure, 2-Hydroxybuttersäure, 3-Hydroxybuttersäure, 4-Hydroxybuttersäure, β-Hydroxyisobuttersäure, Weinsäure, Zitronensäure, oder die verschiedenen Hydroxyfettsäuren in Form ihrer Ester oder Steroide wie Cholsäure, Deoxycholsäure in Form ihrer Ester, Corticosteron, Aldosteron, Östron, Östradiol, Testosteron, oder Ecdyson.

Auch Zucker wie Glucose, Galactose, Mannose, Fucose, Xylose, Arabinose, Altrose, Allose, Rhamnose, Gulose, Idose, Talose, Fructose, Sorbose, Tagatose, Ribose, Desoxyribose, Aminozucker wie N-Acetylneuraminsäure, N-Acetyl-D-Glucosamin, N-Acetyl-D-Galactosamin, N(α-D-Glucopyranosyl)methylamin, D-Glucosamin, (2-Amino-2-desoxy-D-glucose), N-Acetyl-muraminsäure, D-Galactosamin(2-Amino-2-desoxy-D-galactose) oder Disaccharide wie Maltose, Lactose, Chitobiose, Cellobiose oder Oligosaccharide sind als Polyole in allen ihren stereoisomeren Formen (α- oder β-Konfiguration) und allen ihren möglichen Bindungstypen [α-(1,3)-,α-(1,4)-,α-(1,6)-,β-(1,2)-,β-(1,3)-,β-(1,4)-,β-(1,6)-] bei Oligosacchariden für die Abspaltung geeignet.

R¹ bezeichnet in den Verbindungen der Formeln I, II und V CHR⁵R⁶ und R⁵.

Für R⁵ seien Wasserstoff, substituiertes oder unsubstituiertes C₁-C₂₀-Alkyl, C₃-C₂₀-Alkenyl, Arylalkylen oder Aryl genannt.

Als Alkylreste seien verzweigte oder unverzweigte C₁-C₂₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Substituenten kommen ein oder mehrere Substituenten wie Halogen wie Fluor, Chlor oder Brom, Cyano, Nitro, Amino, Hydroxy, Alkyl, Aryl, Alkoxy, Carboxy, Benzyloxy, Phenyl oder Benzyl in Frage.

Als Alkenylreste seien verzweigte oder unverzweigte C₃-C₂₀-Alkenylketten wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl, Dekenyl, Undekenyl, Dodekenyl, Tridekenyl, Tetradekenyl, Pentadekenyl, Hexadekenyl, Heptadekenyl, Octadekenyl, Nonadekenyl oder Eicoxenyl genannt.

Als Substituenten kommen ein oder mehrere Substituenten wie Halogen wie Fluor, Chlor oder Brom, Cyano, Nitro, Amino, Hydroxy, Alkyl, Aryl, Alkoxy, Carboxy, Benzyloxy, Phenyl oder Benzyl in Frage.

Als Arylalkylenreste seien verzweigtkettige oder unverzweigtkettige Phenyl-(C₁-C₅-Alkylen)- oder Naphthyl-(C₁-C₅-Alkylen)-Reste wie Phenylmethylen, Phenylethylen, Phenylpropylen, Phenyl-1-methylethylen, Phenylbutylen, Phenyl-1-methylpropylen, Phenyl-2-methylpropylen, Phenyl-1,1-dimethylethylen, Phenylpentylen, Phenyl-1-methylbutylen, Phenyl-2-methylbutylen, Phenyl-3-methylbutylen, Phenyl-2,2-dimethylpropylen, Phenyl-1-ethylpropylen, Naphthylmethylen, Naphthylethylen, Naphthylpropylen, Naphthyl-1-methylethylen, Naphthylbutylen, Naphthyl-1-methylpropylen, Naphthyl-2-methylpropylen, Naphthyl-1,1-dimethylethylen, Naphthylpentylen, Naphthyl-1-methylbutylen, Naphthyl-2-methylbutylen, Naphthyl-3-methylbutylen, Naphthyl-2,2-dimethylpropylen, oder Naphthyl-1-ethylpropylen, sowie ihre isomeren oder stereoisomeren Formen genannt. Als Arylreste seien substituierte oder unsubstituierte Phenyl- oder Naphthylreste genannt.

Die Arylalkylreste oder Arylreste können gegebenenfalls mit einem oder mehreren Resten wie Halogen wie Fluor, Chlor oder Borm, Cyano, Nitro, Amino, Hydroxy, Alkyl, Alkoxy oder anderen Resten substutiert sein.

Für R⁶ seien Wasserstoff, XH, substituiertes oder unsubstituiertes 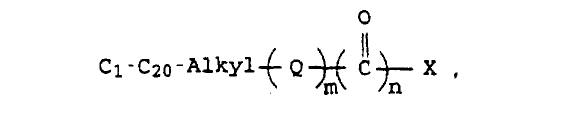 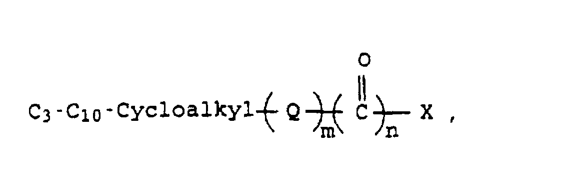 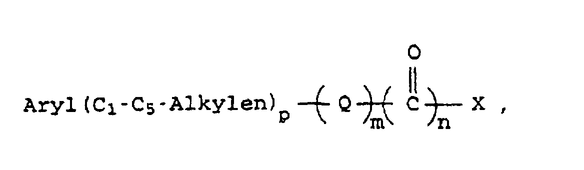 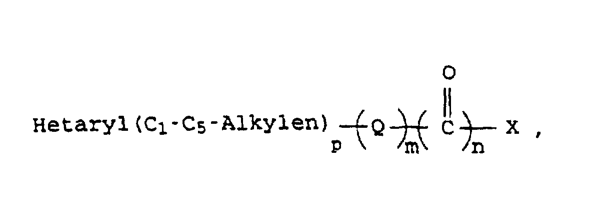 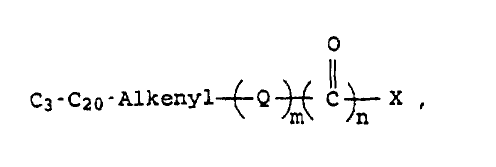 (C₁-C₄Alkyl)₃SiO, Diaryl(C₁-C₅Alkyl)SiO, Aryl(C₁-C₅-Dialkyl)SiO oder R⁷ genannt, wobei Q Sauerstoff oder NH, X Sauerstoff oder NR¹², m, n und p unabhängig voneinander 0 oder 1 bedeuten.

Als Alkylreste seien in der Formel 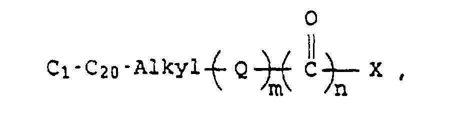 verzweigte oder unverzweigte C₁-C₂₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Cycloalkylreste in der Formel 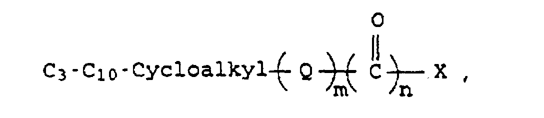 seien verzweigte oder unverzweigte C₃-C₁₀-Cycloalkylketten mit 3 bis 7 Kohlenstoffatomen im Ring oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Die Cycloalkylreste können ggf. mit einem oder mehreren Resten wie Halogen wie Fluor, Chlor oder Brom, Cyano, Nitro, Amino, Hydroxy, Alkyl, Alkoxy oder anderen Resten substituiert sein oder Heteroatome wie S, N und O im Ring enthalten.

Als Arylalkylreste in der Formel 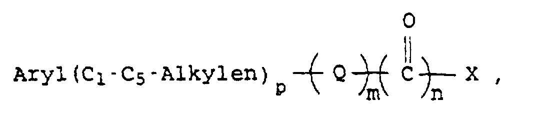 seien verzweigtkettige oder unverzweigtkettige Phenyl-(C₁-C₅-Alkylen) - oder Naphthyl-(C₁-C₅-Alkylen)-Reste wie Phenylmethylen, Phenylethylen, Phenylpropylen, Phenyl-1-methylethylen, Phenylbutylen, Phenyl-1-methylpropylen, Phenyl-2-methylpropylen, Phenyl-1,1-dimethylethylen, Phenylpentylen, Phenyl-1-methylbutylen, Phenyl-2-methylbutylen, Phenyl-3-methylbutylen, Phenyl-2,2-dimethylpropylen, Phenyl-1-ethylpropylen, Naphthylmethylen, Naphthylethylen, Naphthylpropylen, Naphthyl-1-methylethylen, Naphthylbutylen, Naphthyl-1-methylpropylen, Naphthyl-2-methylpropylen, Naphthyl-1,1-dimethylethylen, Naphthylpentylen, Naphthyl-1-methylbutylen, Naphthyl-2-methylbutylen, Naphthyl-3-methylbutylen, Naphthyl-2,2-dimethylpropylen, oder Naphthyl-1-ethylpropylen, sowie ihre isomeren oder stereoisomeren Formen genannt.

Unter Aryl sind beispielsweise Phenyl, Methoxyphenyl oder Naphthyl, oder aromatische Ringe oder Ringsysteme mit 6 bis 18 Kohlenstoffatomen im Ringsystem sowie bis zu 24 weiteren C-Atomen, die weitere nicht aromatische Ringe oder Ringsysteme mit 3 bis 8 C-Atomen im Ring bilden können, zu verstehen, die ggf. mit einem oder mehreren Resten wie Halogen wie Fluor, Chlor oder Brom, Cyano, Nitro, Amino, Hydroxy, Alkyl, Alkoxy oder anderen Resten substituiert sein können. Bevorzugt sind ggf. substituierte Phenyl, Methoxyphenyl und Naphthyl.

Unter Hetaryl(alkylen)reste in der Formel 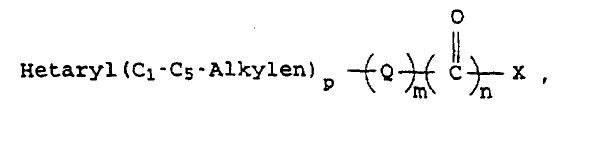 sind Hetarylreste, die einfache oder kondensierte aromatische Ringsysteme mit einem oder mehreren heteroaromatischen 5- bis 7-gliedrigen Ringen, die ein oder mehrere Stickstoff-, Schwefel-und/oder Sauerstoffatome enthalten, zu verstehen, und ggf. mit einer verzweigten oder unverzweigten C₁-C₅-Alkylenkette wie Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen oder 1-Ethylpropylen verbunden sind.

Als Alkenylreste in der Formel 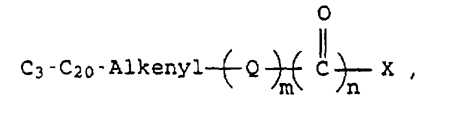 seien verzweigte oder unverzweigte C₃-C₂₀-Alkenylketten wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl,1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl, Dekenyl, Undekenyl, Dodekenyl, Tridekenyl, Tetradekenyl, Pentadekenyl, Hexadekenyl, Heptadekenyl, Octadekenyl, Nonadekenyl oder Eicosenyl genannt.

Als Substituenten der verschiedenen genannten Reste R⁶ kommen ein oder mehrere Substituenten wie Halogen wie Fluor, Chlor oder Brom, Cyano, Nitro, Amino, Hydroxy, Alkyl, Aryl, Alkoxy, Carboxy oder Benzyloxy in Frage.

Als Alkylreste im Rest (C₁-C₄-Alkyl)₃SiO seien verzweigt oder unverzweigte Alkylketten wie Methyl, Ethyl, n-Propyl, 1-Methylethyl, n-Butyl, 1-Methylpropyl, 2-Methylpropyl oder 1,1-Dimethylethyl genannt.

Unter Diaryl in der Formel Diaryl(C₁-C₅-Alkyl)SiO ist beispielsweise Diphenyl, Dinaphthyl oder Phenylnaphthyl zu verstehen, wobei C₁-C₅-Alkyl verzweigte oder unverzweigte Alkylketten wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl oder 1-Ethylpropyl bedeutet.

Als Arylreste im Rest Aryl(C₁-C₅-Dialkyl)SiO seien beispielsweise Phenyl, Methoxyphenyl oder Naphthyl genannt, wobei C₁-C₅-Dialkyl verzweigte oder unverzweigte Alkylketten, die gleich oder verschieden sein können, wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl oder 1-Ethylpropyl bedeutet.

R⁷ bedeutet eine Verbindung der Formel IV 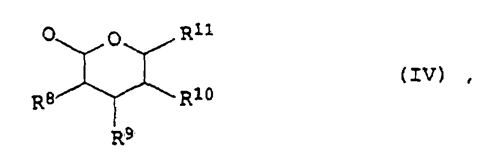 wobei die Substituenten R⁸, R⁹, R¹⁰, R¹¹ folgende Bedeutung haben:
R¹¹ bezeichnet CHR⁵R⁶ und R⁵,
R⁸, R⁹, R¹⁰ unabhängig voneinander, die für die Reste
R², R³, R⁴ im folgenden beschriebene Bedeutung haben und gleich oder verschieden von den Resten
R², R³, R⁴ sein können, oder zwei benachbarte Reste R⁸, R⁹, R¹⁰, R⁶ unabhängig voneinander ein substituiertes oder unsubstituiertes Arylalkylidenacetal oder ein Alkylidenacetal bilden können.

Als Substituenten kommen ein oder mehrere Substituenten wie Halogen wie Fluor, Chlor oder Brom, Cyano, Nitro, Amino, Hydroxy, Alkyl, Aryl, Alkoxy, Carboxy oder Benzyloxy in Frage.

R¹² seien Wasserstoff substituiertes oder unsubstituiertes C₁-C₁₀-Alkyl, C₃-C₈-Cycloalkyl, Aryl, Hetaryl oder Arylalkyl genannt, wobei
- Alkyl verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl n-Heptyl oder n-Octyl, n-Nonyl oder n-Decyl;
- Cycloalkyl verzweigte oder unverzweigte C₃-C₈-Cycloalkylketten mit 3 bis 7 Kohlenstoffatomen im Ring, der ggf. Heteroatome wie S, N oder O enthalten kann, wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-butylcyclopropyl, 1,2-Dimethylcyclopropyl, 1-Methyl-2-Ethylcyclopropyl oder Cyclooctyl;
- Aryl, Phenyl oder Naphthyl;
- Hetaryl einfache oder kondensierte aromatische Ringsysteme mit einem oder mehreren heteroaromatischen 3- bis 7-gliedrigen Ringen,
- Arylalkyl verzweigtkettige oder unverzweigtkettige Phenyl-(C₁-C₅-Alkyl) - oder Naphthyl-(C₁-C₅-Alkyl)-Reste wie Phenylmethyl, Phenylethyl, Phenylpropyl, Phenyl-1-methylethyl, Phenylbutyl, Phenyl-1-methylpropyl, Phenyl-2-methylpropyl, Phenyl-1,1-dimethylethyl, Phenylpentyl, Phenyl-1-methylbutyl, Phenyl-2-methylbutyl, Phenyl-3-methylbutyl, Phenyl-2,2-dimethylpropyl, Phenyl-1-ethylpropyl, Naphthylmethyl, Naphthylethyl, Naphthylpropyl, Naphthyl-1-methylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl, oder Naphthyl-1-ethylpropyl, sowie ihre isomeren oder stereoisomeren Formen bedeutet.

Alle genannten Reste R¹² können gegebenenfalls mit mindestens einem weiteren Rest aus der Gruppe Halogen wie Fluor, Chlor oder Brom, Cyano, Nitro, Amino, Hydroxy oder weiteren Resten substituiert sein.

Für R⁶ sei als Rest außerdem (Aryl)₃CO genannt, wobei Aryl, Phenyl oder Naphthyl bedeutet, daß ggf. mit mindestens einem weiteren Rest wie Halogen wie Fluor, Chlor oder Brom, Cyano, Nitro, Amino, Hydroxy, Alkyl, Aryl, Alkoxy, Carboxy, Benzyloxy substituiert sein kann.

Für R², R³ und R⁴ in den Verbindungen der Formeln I, II oder V seien unabhängig voneinander als Reste Wasserstoff, XH, substituiertes oder unsubstituiertes 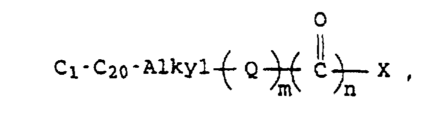 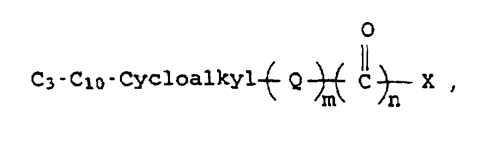 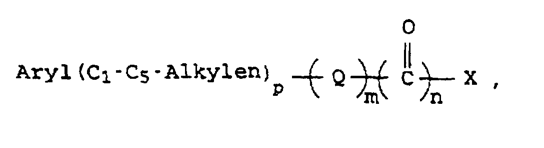 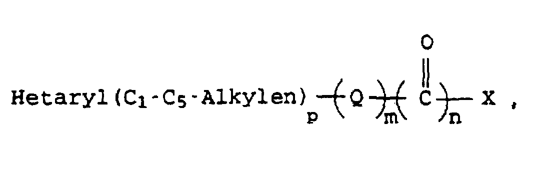 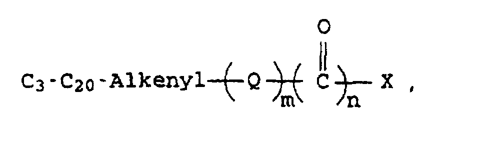 (C₁-C₄Alkyl)₃SiO, Diaryl(C₁-C₅Alkyl)SiO, Aryl(C₁-C₅-Dialkyl)SiO oder R⁷ genannt, wobei Q Sauerstoff oder NH, X Sauerstoff oder NR¹², m, n und p unabhängig voneinander 0 oder 1 bedeuten.

Als Alkylreste seien in der Formel 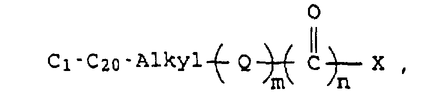 verzweigte oder unverzweigte C₁-C₂₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Cycloalkylreste in der Formel 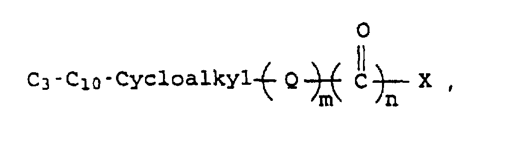 seien verzweigte oder unverzweigte C₃-C₁₀-Cycloalkylketten mit 3 bis 7 Kohlenstoffatomen im Ring oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1- Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Die Cycloalkylreste können ggf. mit einem oder mehreren Resten wie Halogen wie Fluor, Chlor oder Brom, Cyano, Nitro, Amino, Hydroxy, Alkyl, Alkoxy oder anderen Resten substituiert sein oder Heteroatome wie S, N oder O im Ring enthalten.

Als Arylalkylenreste in der Formel 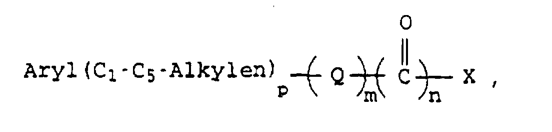 seien verzweigtkettige oder unverzweigtkettige Phenyl- (C₁-C₅-Alkylen) - oder Naphthyl-(C₁-C₅-Alkylen) -Reste wie Phenylmethylen, Phenylethylen, Phenylpropylen, Phenyl-1-methylethylen, Phenylbutylen, Phenyl-1-methylpropylen, Phenyl-2-methylpropylen, Phenyl-1,1-dimethylethylen, Phenylpentylen, Phenyl-1-methylbutylen, Phenyl-2-methylbutylen, Phenyl-3-methylbutylen, Phenyl-2,2-dimethylpropylen, Phenyl-1-ethylpropylen, Naphthylmethylen, Naphthylethylen, Naphthylpropylen, Naphthyl-1-methylethylen, Naphthylbutylen, Naphthyl-1-methylpropylen, Naphthyl-2-methylpropylen, Naphthyl-1,1-dimethylethylen, Naphthylpentylen, Naphthyl-1-methylbutylen, Naphthyl-2-methylbutylen, Naphthyl-3-methylbutylen, Naphthyl-2,2-dimethylpropylen, oder Naphthyl-1-ethylpropylen, sowie ihre isomeren oder stereoisomeren Formen genannt.

Unter Aryl sind beispielsweise Phenyl, Methoxyphenyl oder Naphthyl, oder aromatische Ringe oder Ringsysteme mit 6 bis 18 Kohlenstoffatomen im Ringsystem sowie bis zu 24 weiteren C-Atomen, die weitere nicht aromatische Ringe oder Ringsysteme mit 3 bis 8 C-Atomen im Ring bilden können, zu verstehen, die ggf. mit einem oder mehreren Resten wie Halogen wie Fluor, Chlor oder Brom, Cyano, Nitro, Amino, Hydroxy, Alkyl, Alkoxy oder anderen Resten substituiert sein können. Bevorzugt sind ggf. substituierte Phenyl, Methoxyphenyl und Naphthyl.

Unter Hetarylalkylenreste in der Formel 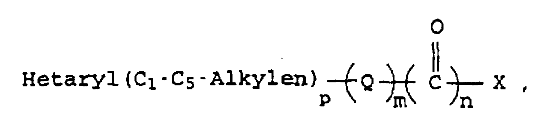 sind Hetarylreste, die einfache oder kondensierte aromatische Ringsysteme mit einem oder mehreren heteroaromatischen 5- bis 7-gliedrigen Ringen, die ein oder mehrere Stickstoff-, Schwefel, und/oder Sauerstoffatome enthalten, zu verstehen, und ggf. mit einer verzweigten oder unverzweigten C₁-C₅-Alkylenkette wie Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen oder 1-Ethylpropylen verbunden sind.

Als Alkenylreste in der Formel 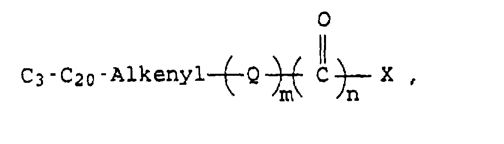 seien verzweigte oder unverzweigte C₃-C₂₀-Alkenylketten wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl, Dekenyl, Undekenyl, Dodekenyl, Tridekenyl, Tetradekenyl, Pentadekenyl, Hexadekenyl, Heptadekenyl, Octadekenyl, Nonadekenyl oder Eicosenyl genannt.

Als Substituenten der verschiedenen genannten Reste R², R³ und R⁴ kommen ein oder mehrere Substituenten wie Halogen wie Fluor, Chlor oder Brom, Cyano, Nitro, Amino, Hydroxy, Alkyl, Aryl, Alkoxy, Carboxy oder Benzyloxy in Frage.

Als Alkylreste im Rest (C₁-C₄-Alkyl)₃SiO seien verzweigt oder unverzweigte Alkylketten wie Methyl, Ethyl, n-Propyl. 1-Methylethyl, n-Butyl, 1-Methylpropyl, 2-Methylpropyl oder 1,1-Dimethylethyl genannt.

Unter Diaryl in der Formel Diaryl(C₁-C₅-Alkyl)SiO ist beispielsweise Diphenyl, Dinaphthyl oder Phenylnaphthyl zu verstehen, wobei C₁-C₅-Alkyl verzweigte oder unverzweigte Alkylketten wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl oder 1-Ethylpropyl bedeutet.

Als Arylreste im Rest Aryl(C₁-C₅-Dialkyl)SiO seien beispielsweise. Phenyl, Methoxyphenyl oder Naphthyl genannt, wobei C₁-C₅-Dialkyl verzweigte oder unverzweigte Alkylketten, die gleich oder verschieden sein können, wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl oder 1-Ethylpropyl bedeutet.

R⁷ bedeutet eine Verbindung der Formel IV 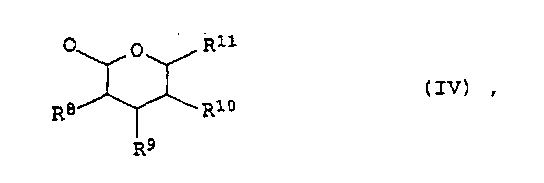 wobei die Substituenten R⁸, R⁹, R¹⁰, R¹¹ und R¹² die oben beschriebene Bedeutung haben.

Für R¹³ in der Formel V seien substituiertes oder unsubstituiertes C₁-C₂₀-Alkyl, C₃-C₂₀-Alkenyl, C₁-C₅-Alkylhetaryl, C₃-C₁₀-Cycloalkyl oder ein- bis viermal verbrücktes substituiertes oder unsubstituiertes C₅-C₂₅-Cycloalkyl, das mit mindestens einem Substituenten ausgewählt aus der Gruppe C₁-C₁₀-Alkyl, C₃-C₁₀-Alkenyl, CO, COO-(C₁-C₁₀-Alkyl), OH, O-(C₁-C₁₀-Alkyl), O-Alkylhetaryl oder O-Alkylaryl substituiert sein kann, oder substituierte oder unsubstituierte Mono-, Di- oder Oligosaccharide oder Hydroxycarbonsäuren genannt, wobei die Reste und Substituenten folgende Bedeutung haben:
- Alkyl verzweigte oder unverzweigte C₁-C₂₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosenyl.
- Alkenyl verzweigte oder unverzweigte C₃-C₂₀-Alkenyl-wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl, Dekenyl, Undekenyl, Dodekenyl, Tridekenyl, Tetradekenyl, Pentadekenyl, Hexadekenyl, Heptadekenyl, Octadekenyl, Nonadekenyl oder Eicosenyl.
- Alkylhetaryl verzweigtkettige oder unverzweigtkettige C₁-C₅-Alkylhetarylreste, die ein oder mehrere Stickstoff-, Schwefel- und/oder Sauerstoffatome im Ring oder Ringsystem enthalten,
- Cycloalkyl verzweigte oder unverzweigte C₃-C₁₀-Cycloalkylketten mit 3 bis 7 Kohlenstoffatomen im Ring, der Heteroatome wie S, N oder O enthalten kann, oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl oder
- Cycloalkyl substituierte oder unsubstituierte verzweigte oder unverzweigte C₅-C₂₅-Cycloalkylketten, die ein- bis viermal verbrückt sein können, und mit mindestens einem Substituenten ausgewählt aus der Gruppe C₁-C₁₀-Alkyl, C₃-C₁₀-Alkenyl, CO, COO-(C₁-C₁₀-Alkyl), OH, O-(C₁-C₁₀-Alkyl), O-Alkylhetaryl oder O-Alkylaryl substituiert sein können, wobei
- Alkyl verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl,
- Alkenyl verzweigte oder unverzweigte C₃-C₈-Alkenylketten wie beispielsweise Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl
- Alkylhetaryl verzweigtkettige oder unverzweigtkettige C₁-C₅-Alkylhetarylreste, die ein oder mehrere Stickstoff-, Schwefel- und/oder Sauerstoffatome im Ring oder Ringsystem enthalten,
- Alkylaryl verzweigtkettige oder unverzweigtkettige C₁-C₆-Alkyl-phenyl- oder C₁-C₆-Alkyl-naphthylreste wie Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Pentylphenyl, 1-Methylbutylphenyl, 2-Methylbutylphenyl, 3-Methylbutylphenyl, 2,2-Dimethylpropylphenyl, 1-Ethylpropylphenyl, n-Hexylphenyl, 1,1-Dimethylpropylphenyl, 1,2-Dimethylpropylphenyl, 1-Methylpentylphenyl, 2-Methylpentylphenyl, 3-Methylpentylphenyl, 4-Methylpentylphenyl, 1,1-Dimethylbutylphenyl, 1,2-Dimethylbutylphenyl, 1,3-Dimethylbutylphenyl, 1,2-Dimethylbutylphenyl, 1,3-Dimethylbutylphenyl, 2,2-Dimethylbutylphenyl, 2,3-Dimethylbutylphenyl, 3,3-Dimethylbutylphenyl, 1-Ethylbutylphenyl, 2-Ethylbutylphenyl, 1,1,2-Trimethylpropylphenyl, 1,2,2-Trimethylpropylphenyl, 1-Ethyl-1-methylpropylphenyl, 1-Ethyl-2-methylpropylphenyl, Methylnaphthyl, Ethylnaphthyl, Propynaphthyl, 1-Methylethylnaphthyl, Butylnaphthyl, 1-Methylpropylnaphthyl, 2-Methylpropylnaphthyl, 1,1-Dimethylethylnaphthyl, Pentylnaphthyl, 1-Methylbutylnaphthyl, 2-Methylbutylnaphthyl, 3-Methylbutylnaphthyl, 2,2-Dimethylpropylnaphthyl, 1-Ethylpropylnaphthyl, n-Hexylnaphthyl, 1,1-Dimethylpropylnaphthyl, 1,2-Dimethylpropylnaphthyl, 1-Methylpentylnaphthyl, 2-Methylpentylnaphthyl, 3-Methylpentylnaphthyl, 4-Methylpentylnaphthyl, 1,1-Dimethylbutylnaphthyl, 1,2-Dimethylbutylnaphthyl, 1,3-Dimethylbutylnaphthyl, 1,2-Dimethylbutylnaphthyl, 1,3-Dimethylbutylnaphthyl, 2,2-Dimethylbutylnaphthyl, 2,3-Dimethylbutylnaphthyl, 3,3-Dimethylbutylnaphthyl, 1-Ethylbutylnaphthyl, 2-Ethylbutylnaphthyl, 1,1,2-Trimethylpropylnaphthyl, 1,2,2-Trimethylpropylnaphthyl, 1-Ethyl-1-methylpropylnaphthyl, 1-Ethyl-2-methylpropylnaphthyl,
   in den genannten Substituenten der C₅-C₂₅-Cycloalkylketten vorzugsweise bedeutet.
   Unter C₅-C₂₅-Cycloalkylketten sind bevorzugt Stereoide wie Cholsäure, Deoxycholsäure in Form ihrer Ester, Corticosteron, Aldosteron, Östron, Östradiol, Testosteron, oder Ecdyson zu verstehen.
- Mono-, Di- oder Oligosaccharide wie Glucose, Galactose, Mannose, Fucose, Xylose, Arabinose, Altrose, Allose, Rhamnose, Gulose, Idose, Talose, Fructose, Sorbose, Tagatose, Ribose, Desoxyribose oder Aminozucker wie N-Acetylneuraminsäure, N-Acetyl-D-Glucosamin, N-Acetyl-D-Galatosamin, N(α-D-Glucopyranosyl)methylamin, D-Glucosamin(2-Amino-2-desoxy-D-glucose), N-Acetylmuraminsäure, D-Galactosamin(2-Amino-2-desoxy-D-galactose) oder Disaccharide wie Maltose, Lactose, Chitobiose, Cellobiose oder Oligosaccharide in allen ihren stereoisomeren Formen (α- oder β-Konfiguration) und allein ihren möglichen Bindungstypen[α-(1,3)-, α-(1,4)-, α-(1,6)-, β-(1,2)-, β-(1,3)-, β-(1,4)-, β-(1,6)] als Homo-oder Heteromere bei Oligosacchariden können glycosidisch gebunden sein.
- Hydroxycarbonsäuren wie Milchsäure, Mandelsäure, Pantoinsäure, 3-Phenylmilchsäure, 2-Hydroxybuttersäure, 3-Hydroxybuttersäure, 4-Hydroxybuttersäure, β-Hydroxyisobuttersäure, Weinsäure, Zitronensäure, Serin, Threonin oder die verschiedenen Hydroxyfettsäuren in Form ihrer Ester.

Alle genannten Reste und Substituenten sind falls erforderlich für die Synthese mit Schutzgruppen zu versehen.

Das erfindungsgemäße Verfahren zur Herstellung der Kohlenhydratderivate wird vorteilhafterweise in einer Reaktionssequenz durchgeführt bei der zunächst die feste Phase mit dem bevorzugten Linker der Formel VI 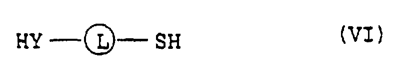 geeignet funktionalisiert wird. Die Funktionalisierung von Chlormethylpolystyrol [= Merrifield-Harz = (1)], das mit 1 % Divinylbenzol vernetzt wurde, wird beispielsweise wie folgt durchgeführt: 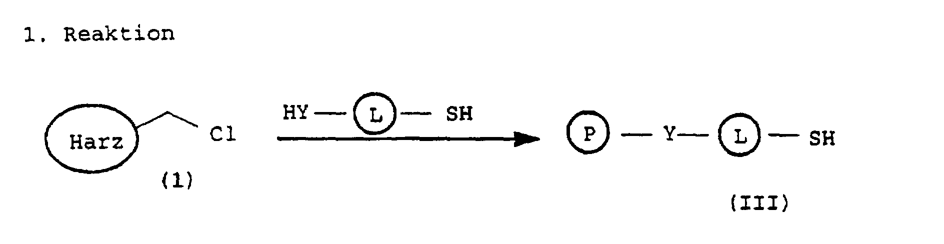 Reaktion (1) wird in Gegenwart einer Base, bevorzugt tert.-Aminbasen wie (iPr)₂NEt, NEt₃ oder DBU, durchgeführt. Weitere geeignete Basen sind Kalium- oder Natriumcarbonat oder Natriumhydrid oder Natriumhexamethyldisilasan, bevorzugt sind DBU oder Natriumhydrit. Als Lösungsmittel sind beliebige aprotische Lösungsmittel, beispielsweise DMF, THF, CH₂Cl₂ oder deren Gemische, zu nennen. Die Reaktion wird in einem Temperaturbereich von +10 bis 70°C, bevorzugt +10 bis +40°C durchgeführt.

An die funktionalisierte feste Phase wird anschließend in Gegenwart eines Promotors ein erster Zucker angekoppelt, der über eine geeignete Gruppe Z wie beispielsweise N³, Halogen, 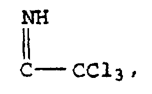 OCH₂CH₂CH₂CH=CH₂, OPO₂H₂, S-Aryl, S-Alkyl aktiviert wurde (2. Reaktion).

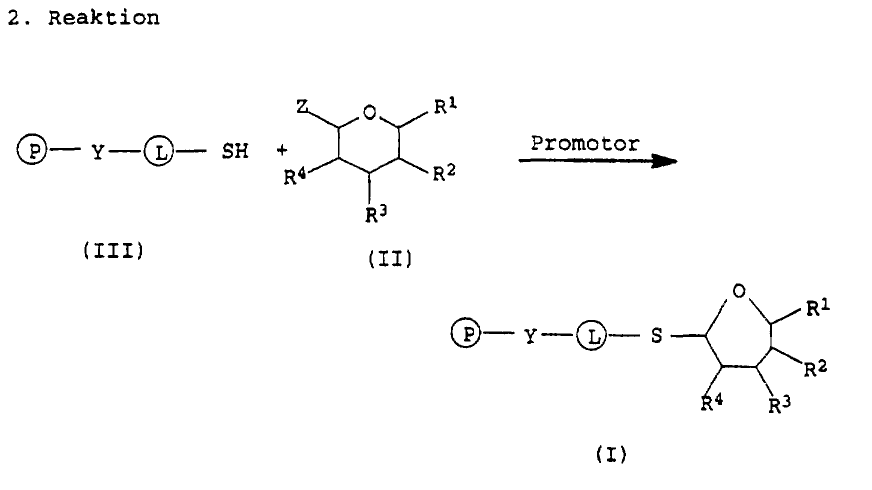

Als Promotoren eignen sich beispielsweise Lewissäuren wie BF₃·Et₂O, TMSOTf, TESOTf oder elektrophile Reagentien wie J₂, JCl, JBr oder thiophile Reagentien wie DMTST oder schwerlösliche Halogensalze bildende Schwermetallsalze wie AgOTf, Hg(Cn)₂ oder Hg(OTfa)₂, bevorzugt sind Lewissäuren. Weitere geeignete Promotoren sowie geeignete Glycosyldonoren mit den verschiedenen möglichen Fluchtgruppen Z sind Waldmann H. (Nachr. Chem. Tech. Lab. 39, 1991:675-682), Sinay P. (Pure Appl. Chem. 63, 1991:519-528) oder Schmidt R.R. (Angew. Chem. 98, 1986: 213-236) zu entnehmen. Die Reaktion wird zwischen -100°C und +100°C, bevorzugt zwischen -60°C und +80°C, ganz besonders bevorzugt zwischen -20°C und +40°C durchgeführt. Als Lösungsmittel sind beliebige aprotische hydroxygruppenfreie organische Solventien wie CH₂Cl₂, Toluol, Acetonitril oder deren Gemische geeignet; eventuell unter Zusatz wasserentziehender Reagentien wie Molsiebe.

Das durch die Anknüpfung des ersten Zuckers an die feste Phase entstandene Thioglycosid kann in verschiedener Weise wie folgt beschrieben weiter modifiziert bzw. umgesetzt werden.

Die Schutzgruppen an den verschiedenen Hydroxy- oder Hydroxymethylgruppen des Zuckers können ganz oder teilweise abgespalten werden und das mit mindestens einer freien Hydroxy- oder Hydroxymethylgruppe versehene Thioglycosid kann mit den unter R¹ bis R⁴ bzw. R⁸ bis R¹¹ genannten Resten oder mit Verbindungen der Formel II umgesetzt werden. Dabei können gleiche oder verschiedene Reste oder gleiche oder verschiedene Verbindungen der Formel II mit den Hydroxy- oder Hydroxymethylgruppen zur Reaktion gebracht werden, wobei verschiedene Reste oder Verbindungen der Formel II sequentiell mit dem Kohlenhydrat umgesetzt werden können oder aber Gemische aus den Komponenten. Die Umsetzung erfolgt dabei vorzugsweise mit Acylierungs-, Carbamoylierungs- oder Alkylierungsreagentien. Die freien Hydroxy- oder Hydroxymethylgruppen können mit mehr, gleich oder weniger als einem molaren Äquivalent der Reste oder der Verbindungen der Formel II umgesetzt werden, so daß vollständiger oder teilweiser Umsatz des Thioglycosid erreicht wird. Anschließend kann das Kohlenhydrat unter Einführung eines weiteren Substituenten von der festen Phase abgespalten werden oder aber ohne Einführung eines weiteren Substituenten abgespalten werden, so daß eine freie Hydroxygruppe entsteht.

Im Falle, daß das Thioglycosid mit mindestens einem weiteren Kohlenhydrat umgesetzt wurde, kann ein weiterer Zyklus mit den oben beschriebenen Variationen als Möglichkeit durchlaufen werden. Durch vielfaches Durchlaufen des Synthesezyklusses lassen sich komplexe Kohlenhydrate aufbauen.

Bevorzugt wird der Synthesezyklus durch Entschützen des Restes R¹ oder bei fortgeschrittener Synthese des Restes R¹¹ und damit an der freien Hydroxymethylgruppe durchgeführt (Reaktion 3). 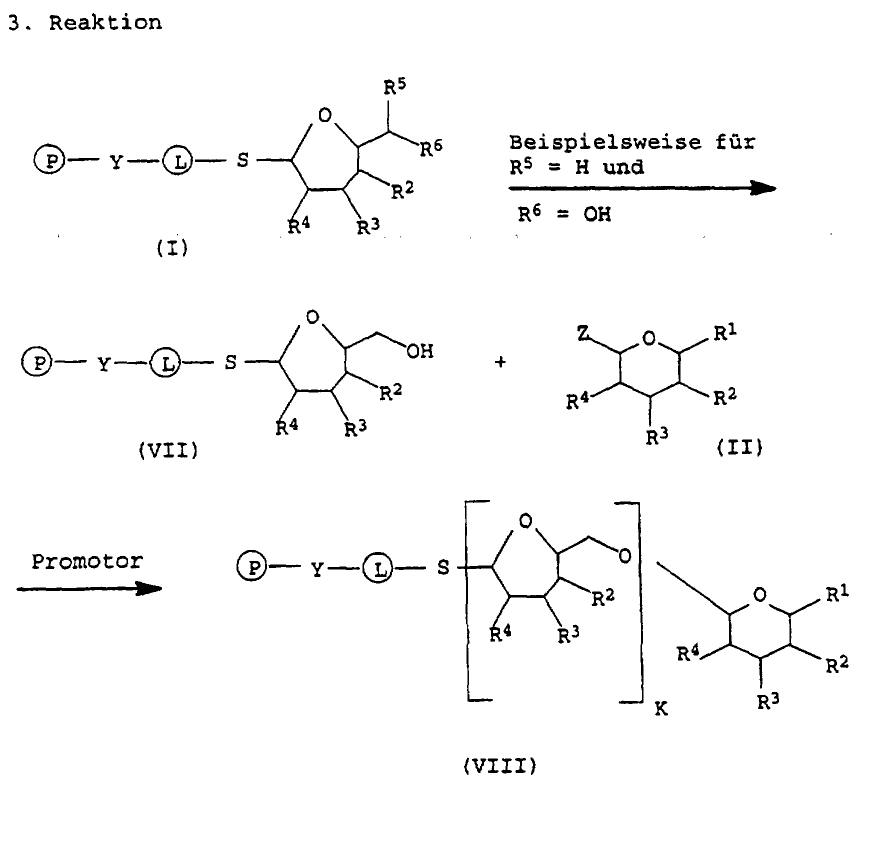 Zum Entschützen wird vorzugsweise eine Umesterung mit Alkoholaten wie NaOMe oder eine Verseifung z.B. mit Hydrazin in Gegenwart eines Lösungsmittels verwendet. Als Lösungsmittel eignen sich aprotische, polare oder unpolare Lösungsmittel oder deren Gemische. Die Reaktion wird zwischen 0°C bis 120°C, bevorzugt 0°C bis 60°C durchgeführt. Anschließend wird die entstehende Verbindung VII mit einem weiteren Zucker der Formel II wie oben beschrieben, umgesetzt. Die angegebenen Reste R¹ bis R⁴ in den Formeln I bzw. VII und II können gleich oder verschieden sein.

Der Reaktionszyklus kann beliebig oft durchlaufen werden, so daß Polysaccharide mit großen K-Werten (K>10) synthetisiert werden können. Anschließend kann das Polysaccharid von der festen Phase wie beschrieben unter Einführung eines weiteren Substituenten oder unter Bildung der freien Hydroxygruppe abgespalten werden.

Verbindungen der Formel I eignen sich auch nach Abspaltung mindestens einer Schutzgruppe für eine weitere Enzym katalysierte Synthese an Kohlenhydraten und/oder zum Aufbau von komplexen Kohlenhydraten.

So können beispielsweise Proteasen, Lipasen und/oder Esterasen je nach Synthesebedigungen zur Knüpfung von Ester- oder Amidbindungen oder zur Spaltung entsprechender Bindung genutzt werden. Mit Glycosyltransferasen oder Glycosidasen können weitere Zuckerreste an das Thioglycosid angeknüpft werden. Eine Kombination der chemischen und enzymatischen Synthese ist möglich.

Für das erfindungsgemäße Verfahren sind prinzipiell alle aus der Zuckersynthese bekannten Schutzgruppen wie Acetyl, Benzoyl, Pivaloyl, Benzyl, 4-Methoxybenzoyl, tert.-Butyldimethylsilyl, Phenylditert.butyl, Trimethylsilyl, Benzyliden, 4-Methoxybenzyliden, Alloxicarbonyl, Propenyl, 4-Pentenyl geeignet. Weiter für das Verfahren nutzbare Schutzgruppen sind von Greene T.W. und Wats P.G.M. in Protective Groups in Organic Synthesis (John Wiley & Sons, Inc., 1991) beschrieben. Besonders erwähnt seien die dort beschriebenen folgenden Schutzgruppen für Hydroxygruppen (Seite 10ff), für Carbonylgruppen (Seite 175ff), für Carboxylgruppen (Seite 224ff), für Aminogruppen (Seite 309ff) sowie weitere nutzbare Schutzgruppen (Seite 406ff),

Das erfindungsgemäße Verfahren kann in einer Reihe paralleler automatisierter Syntheseansätze durchgeführt werden. Auch Reaktantengemische können in einem Syntheseansatz oder parallelen Syntheseansätzen eingesetzt werden.

Das erfindungsgemäße Verfahren eignet sich sehr gut zur Erzeugung einer großen Zahl strukturell vielfältiger Verbindungen der Formeln I und V, da die Substituenten R¹ bis R¹³ unabhängig voneinander auf einfache Weise breit variierbar sind.

Im Vergleich zu Reaktionen in Lösung weisen die Umsetzungen am polymeren Träger große Vorteile auf. So findet man in den Produkten erheblich weniger Verunreinigungen, so daß eine chromatographische Auftrennung nicht erforderlich ist. Die guten Ausbeuten, die hohe Reinheit der abgespaltenen Produkte und die einfache Reaktionsführung des erfindungsgemäßen Verfahrens machen seine Anwendung im Rahmen der kombinatorischen Synthese sehr attraktiv. Besonders vorteilhaft bei diesem Verfahren ist beispielsweise, daß auf die Verwendung teurer Polymere verzichtet werden kann, da ein kostengünstiger Linker zur Funktionalisierung an beliebige feste Phasen angebunden werden kann.

Das Verfahren eignet sich auch besonders gut zur Herstellung definierter Gemische von Kohlenhydratderivaten der Formel I. Dazu geht man nicht von einer Einzelsubstanz aus, die an die feste Phase gebunden wird, sondern bindet ein Gemisch, bevorzugt ein nach Stöchiometrie und Substanzen bekanntes Gemisch, an die feste Phase.

Der festphasengebundene Reaktionspartner wird dann gemäß dem beschriebenen Verfahren mit anderen Reaktionspartnern umgesetzt.

Der Vorteil dieser Festphasensynthese liegt in der schnellen Erzeugung einer Vielzahl von einzelnen Verbindungen, die anschließend auf ihre Wirksamkeit in Testsystemen untersucht werden können. Diese Vielzahl von einzelnen Verbindungen bilden sog. Substanzbibliotheken.

Zur Testung können die Substanzgemische entweder vorher aufgetrennt werden oder direkt in Form der Gemische eingesetzt werden. Im zweiten Fall erfolgt eine Identifizierung eines potentiellen Wirkstoffes nach der Testung.

Ein weiterer Gegenstand der Erfindung ist die Verwendung des erfindungsgemäßen Herstellverfahrens für gebundene oder freie Kohlenhydratderivate der Formeln I oder V zur Generierung von Substanzbibliotheken.

Hierunter ist sowohl die oben beschriebene Erzeugung von Kohlenhydratgemischen als auch die Herstellung einer Vielzahl von Einzelsubstanzen der Formeln I oder V, beispielsweise durch paralleles Ausführen vieler gleichartiger Reaktionen, bei der jeweils ein Reaktionspartner verändert wurde, zu verstehen.

Das parallele Ausführen vieler gleichartiger Reaktionen erlaubt auf schnelle Weise die systematische Variation aller funktionellen Gruppen in den Formeln I oder V.

Die so erzeugbaren Substanzbibliotheken können im sogenannten Massenscreening schnell auf eine bestimmte Wirksamkeit überprüft werden. Dadurch wird die Suche nach potenten Wirkstoffen stark beschleunigt.

Ein weiterer Gegenstand der Erfindung sind trägergebundene Kohlenhydratderivate der allgemeinen Formel I. Diese Verbindungen sind herstellbar, indem man das obengenannte Herstellverfahren ohne eine Abspaltung der erhaltenen Kohlenhydrate der Formel I von der festen Phase ausführt.

Dadurch bleiben die Kohlenhydratderivate an die feste Phase gebunden und können als solche leicht in Testverfahren, bevorzugt in in-vitro Testsystemen, eingesetzt werden.

Der Vorteil der trägergebundenen Kohlenhydratderivate liegt in ihrer leichten Handhabbarkeit. Beispielsweise können sie leicht aus der Reaktionslösung durch Filtration oder Zentrifugation isoliert werden.

Zusätzlich ist die Identifizierung eines Wirkstoffs erheblich erleichtert, da die trägergebundenen Kohlenhydratderivate schon vereinzelt vorliegen und so eine Trennung entfällt.

Die folgenden Beispiele dienen der weiteren Veranschaulichung der Erfindung, ohne sie in irgendeiner Weise einzuschränken.

### Beispiel 1

### 1. Synthese des Glycosyldonors

1,6-Di-O-acetyl-2,3,4-tri-O-benzyl-α,β-D-glucopyranosid (1) 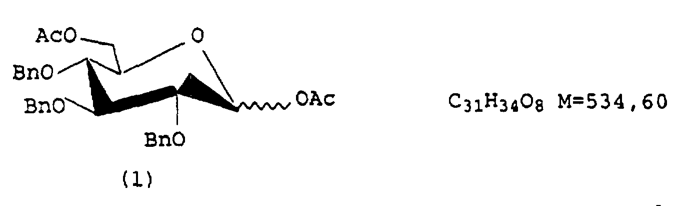 Verbindung 1 wurde nach einer Literaturvorschrift von C. Schuerch et al. [(Carbohydr. Res. 73 (1979), 273-276)] dargestellt.

6-O-Acetyl-2,3,4-tri-O-benzyl-α,β-D-glucopyranose (2) 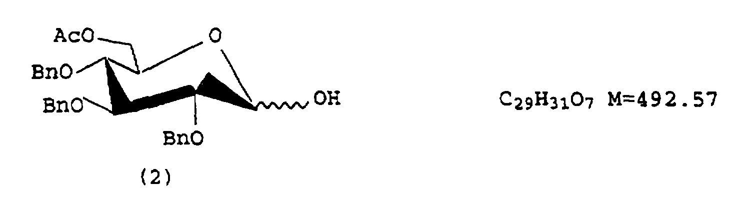 5 g (9,35 mmol) von Verbindung 1 werden in 20 ml Dimethylformamid gelöst. Es werden 0,95 g Hydraziniumacetat (10,28 mmol) hinzugegeben und man erwärmt 20 min auf 50°C. Das Hydraziniumacetat geht vollständig in Lösung, dünnschichtchromatographische Kontrolle (PE=Petrolether / EE=Essigester 2:1) zeigt vollständige Reaktion an. Die Reaktionslösung wird auf 20 ml Wasser gegeben und dreimal mit je 50 ml Diethylether extrahiert. Die vereinigten organischen Phasen werden über Magnesiumsulfat getrocknet und im Vakuum bei 50°C Badtemperatur eingeengt. Das erhaltene Produkt (4,33 g, 94 %) kann ohne weitere Reinigung umgesetzt werden. Die physikalischen Daten stimmen mit denen der Literatur (Carbohydr. Res. 191 (1) (1989) 21-28) überein.

O-(6-O-Acetyl-2,3,4-tri-O-benzyl-D-glucopyranosyl)-trichloracetimidat (3)

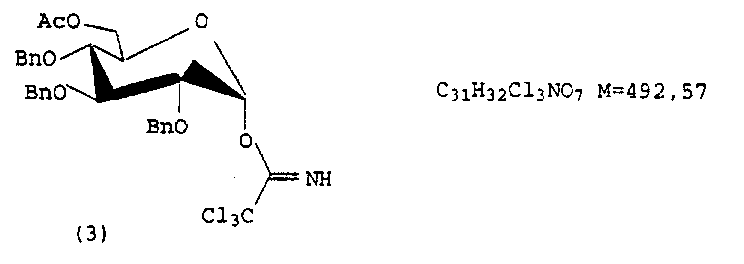

Verbindung 2 (4,3 g 8,7 mmol) wird bei 23°C zusammen mit 3,5 ml (34 mmol) Trichloracetonitril in 20 ml Dichlormethan gelöst. Sodann werden 0.1 ml 1,8-Diazabicyclo[5.4.0]undec-7-en hinzugefügt. Nach 30 min zeigt das Dünnschichtchromatogramm (PE/EE 4:1) die vollständige Reaktion an. Die Reaktionslösung wird im Vakuum eingeengt und über eine kurze Kieselgelsäule mit PE/EE 2:1 filtriert, wobei dem Eluens 1 % Triethylamin zugesetzt wird. 4,88 g (7,65 mmol, 88 %) Produkt wurde erhalten.

¹H-NMR (250 MHz): δ 2,0 (s, 3 H, OAc), 3,60 (dd, ³J_{2,3} = 9,5 Hz, ³J_{3,4} = 9,5 Hz, 1 H, 3-H), 3,74 (dd, ³J_{1,2} = 3,5 Hz, ³J_{2,3} = 9,5 Hz, 1 H, 2-H), 4,05 (m, 1 H, 5-H), 4,07 (d, ³J_{3,4} = 9,5 Hz, ³J_{4,5} = 9,5 Hz, 1 H, 4-H), 4,15-4,35 (m, 2 H, 2 6-H), 4,55-5,10 (m, 6 H, CH₂Ph), 6,46 (d, ³J_{1,3} = 3,5 Hz, 1 H, 1-H), 7,15-7,45 (m, 6 H, Phenyl, 8,61 (s, 1 H, NH).

### Beispiel 2

### 2. Funktionalisierung des Synthesepolymers

### a) ohne Schutzgruppe

Divinylbenzol-Polystyrol-Copolymer funktionalisiert mit Propan-1,3-dithiol (4) 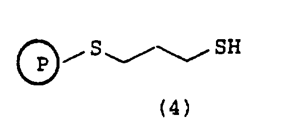 1 g Chlormethyliertes, 1 %-Divinylbenzol-Polystyrol-Copolymer der Fa. Fluka wird in 10 ml Toluol gequollen, 1 ml Propan-1,3-dithiol (10 mmol) werden hinzugefügt und durch Umschwenken vermischt. Nach 15 min tropft man 0,45 ml (3 mmol) 1,8-Diazabicyclo[5.4.0]-undec-7-en dazu und läßt die Reaktion unter gelegentlichem Umschwenken 24 h bei 23°C stehen. Das Harz wird abfiltriert und man wäscht mehrmals abwechselnd mit Dichlormethan und Dimethylformamid. Danach wird 12 h bei 90°C im Vakuum getrocknet. Durch Elementaranalyse wird die Funktionalisierung zu 0,5 mmol/g bestimmt.

### b) mit Schutzgruppe

1-Dimethoxytritylthio-propan-3-ol (5) 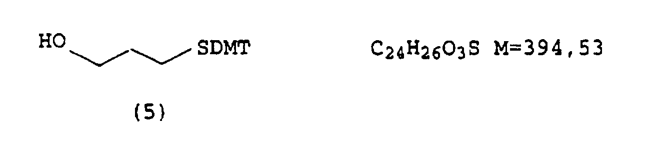 6,77 g Dimethoxytriphenylmethylchlorid (20 mmol) werden in 40 ml Pyridin gelöst. Bei 10°C gibt man 2 ml (18 mmol) 3-Mercaptopropionsäuremethylester hinzu und läßt 14 h bei 23°C rühren. Die Reaktionslösung wird dann mit 150 ml Diethylether verdünnt und mit 20 ml Wasser extrahiert. Die organische Phase wird nach dem Trocknen mit Magnesiumsulfat im Vakuum eingeengt und mehrmals mit Toluol coevaporiert. Das so erhaltene Rohprodukt wird in 40 ml DMF gelöst. Bei 0°C werden 820 mg Lithiumaluminiumhydrid (21,6 mmol) portionsweise hinzugegeben. Nach 2 h ist die Reaktion abgeschlossen (Toluol/EE 5:1). Zur Aufarbeitung verdünnt man mit 10 ml Essigester, nach 15 min mit 100 ml Diethylether und sodann langsam mit 80 ml Wasser. Nach 1 h Rühren filtriert man über Kieselgur ab und wäscht mehrmals mit Diethylether. Die organische Phase wird nach dem Trocknen eingeengt und durch zweifache Kristallisation aus PE/EE (1:1) erhält man 6,1 g von Verbindung 5 (86 %).

¹H-NMR (250 Mhz, CDCl₃): δ = 1,25 (bs, 1 H, OH), 1,64 (tt, ³J = 6,65 Hz, 2 H, CH₂), 2,28 (t, ³J = 7,2 Hz, 2 H, CH₂), 3,58 (dt, ³J_{OH,CH} = 5,7 Hz, ³J_{CH,CH} = 6,65 Hz, 2 H, CH₂) 3,79 (s, 6 H, OCH₃), 6,7-7,4 (m, 13 H, Phenyl).

Divinylbenzol-Polystyrol-Copolymer funktionalisiert mit Propan-3-ol-1-thiol (6) 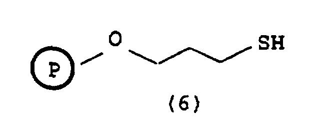 0,5 g Chlormethyliertes, 1 %-Divinylbenzol-Polystyrol-Copolymer wird in 5 ml Tetrahydrofuran gequollen und mit 632 mg (1,6 mmol) 1-Dimethoxytritylthio-propan-3-ol (5) 380 µl (1,92 mmol) 15-Krone-5 (= 1,4,7,10,13-Pentaoxacyclopentadecan) und 46 mg (1,92 mmol) Natriumhydrid versetzt. Man erhitzt 24 h lang auf 60°C und arbeitet dann wie bei Verbindung 4 beschrieben auf. Anschließend wird mit einer 5 %-Lösung von Trifluoressigsäure in Dichlormethan gewaschen bis die Waschlösung farblos bleibt. Nach Waschen mit Dichlormethan und Dimethylformamid wird 12 h bei 90°C getrocknet. Die Elementaranalyse ergibt eine Funktionalisierung von 0,5 mmol/g.

### Beispiel 3

### 3. Vorschrift zur Festphasenglycosylierung

Glycosyliertes, vollgeschütztes Synthesepolymer (7); n=1.5 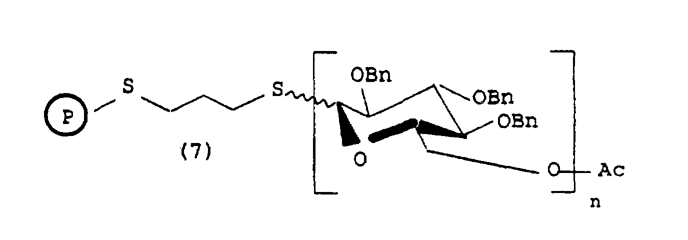 200 mg des Syntheseharzes 4 werden unter Schutzgas in eine Glaskartusche (8x80 mm) gefüllt und mit Teflonstopfen verschlossen. Man löst 191 mg (0,3 mmol) der Verbindung 3 in Dichlormethan; diese Lösung wird in das Reaktionsgefäß eingespritzt und für 10 min unter gelegentlichem Umschwenken stehen gelassen. Dann gibt man 100 µl einer 0,5 M Lösung von Trimethylsilyltriflat in Dichlormethan hinzu und schüttelt das Reaktionsgefäß in waagrechter Lage für 1 h. Es wird anschließend mit Dichlormethan und Dichlormethan/Acetonitril 1:1 abwechselnd gewaschen und im Vakuum getrocknet. Die Vollständigkeit der Glycosylierungsreaktion wird mit MALDI-TOF-MS kontrolliert. Dazu werden 2 mg des Harzes 7 15 min lang mit 0,1 ml einer frisch bereiteten Lösung von Dimethyl (methylthio)sulfonium-tetrafluorborat-Lösung (DMTSB) in Dichlormethan/Methanol 9:1 (10 mg pro ml) behandelt. 10 µl dieser Suspension werden sodann mit dem gleichen Volumen einer Lösung von 2,5-Dihydroxybenzoesäure in Acetonitril vermischt. Dieser Mischung entnimmt man 0,8 µl, die direkt auf das Target des Massenspektrometers aufgetragen und nach 5 min Trocknen gemessen wird.

### Beispiel 4

### 4. Vorschrift zur Desacetylierung am Syntheseharz

Glycosyliertes, 6-O-desacetyliertes Syntheseharz (8); n=1-5 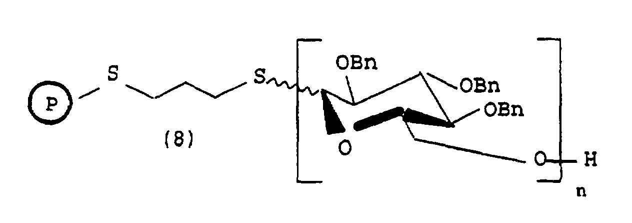 Das glycosylierte Syntheseharz 7 (0,1 mmol) wird 2 ml Dichlormethan und 0,2 ml einer 0,5 M Lösung von Natriummethanolat in Methanol versetzt. Das Reaktionsgefäß wird 2 h geschüttelt. Man wäscht mit Dichlormethan und schüttelt dann 20 min mit einer Lösung von 2 Äquivalenten 15-Krone-5 in Dichlormethan/Methanol 20:1. Es wird erneut mit Dichlormethan sowie Dichlormethan/Acetonitril 1:1 gewaschen und im Vakuum getrocknet. Die Reaktionskontrolle wird wie bei Syntheseharz 7 durchgeführt.

### Beispiel 5

### 5. Abspaltung der Oligosaccharide vom glycosylierten Synthesepolymer

1-O-Methyl-α,β-D-Glucopyranosyl-(1→6) -oligosaccharid (9); n=2-5

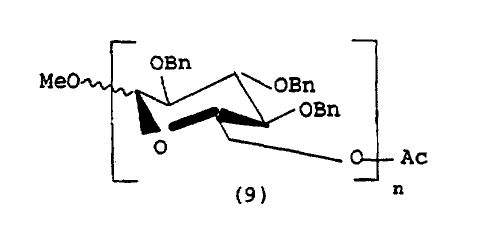

Das glycosylierte Syntheseharz 7 (ca. 0,05 mmol, das Gewicht variiert mit n) wird in 4 ml Dichlormethan/Methanol 9:1 gequollen und mit 13 mg (0,1 mmol) Dimethyl- (methylthio) -sulfonium-tetrafluorborat und 13 µl Diisopropylethylamin (0,1 mmol) versetzt. Nach 2 h filtriert man vom Polymer ab und wäscht mit Dichlormethan sowie Dichlormethan/Acetonitril (1:1). Die gesammelten Filtrate werden mit 60 ml Diethylether verdünnt und zweimal mit 15 ml Wasser extrahiert. Die getrocknete organische Phase wird eingeengt und über Kieselgel (Toluol/EE 6:1) gereinigt.

Es werden die anomeren Mischungen für n=2-5 jeweils ohne massenspektroskopisch nachweisbare Verunreinigungen von länger- oder kürzerkettigen Zuckern erhalten. Das Anomerenverhältnis liegt für jeden Glycosylierungsschritt bei α,β ≈ 1.

¹H-NMR (600 Mhz, CDCl₃) : δ in ppm = 4,95-5,02 H-1α, 4,25-4,30 H1-β. ¹³C-NMR (150,9 Mhz, CDCl₃) δ: 97-99 C-1α, 104-106 C1-β.

Allgemeine Formel für Verbindung 9: C₂₇ₙ₊₃H₂₈ₙ₊₆O₅ₙ₊₂ M=432,515n+74,08

## Patentansprüche

1. Verfahren zur Herstellung von Kohlenhydratderivaten der Formel I, 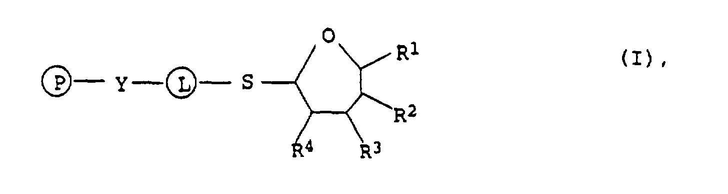 in der die Variable und Substituenten folgende Bedeutung haben:
(P) eine feste Phase
(L) ein aliphatischer Linker mit 2 bis 12 Kohlenstoffatomen,
R¹ CHR⁵R⁶, R⁵
R², R³, R⁴ unabhängig voneinander Wasserstoff, XH, substituiertes oder unsubstituiertes 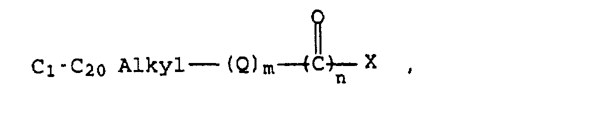 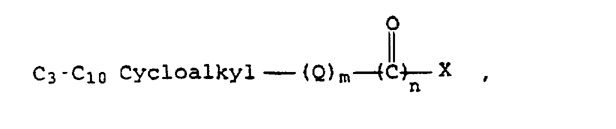 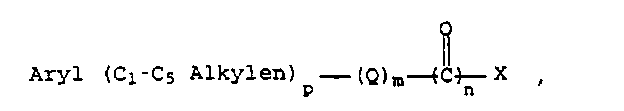 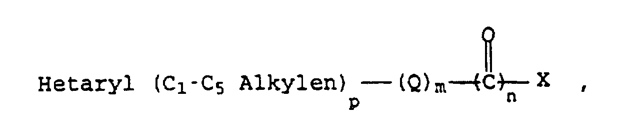 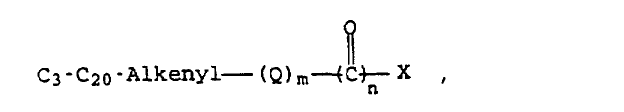 (C₁-C₄-Alkyl)₃SiO, Diaryl(C₁-C₅-Alkyl)SiO, Aryl(C₁-C₅-Dialkyl)SiO oder R⁷ und m = 0,1; n = 0,1; p = 0,1
oder zwei benachbarte Reste R², R³, R⁴, R⁶ unabhängig voneinander ein substituiertes oder unsubstituiertes Arylalkylidenacetal oder ein Alkylidenacetal bilden,
R⁵ Wasserstoff, substituiertes oder unsubstituiertes C₁-C₂₀-Alkyl, C₃-C₂₀ Alkenyl, Arylalkylen oder Aryl,
R⁶ Wasserstoff, XH, substituiertes oder unsubstituiertes 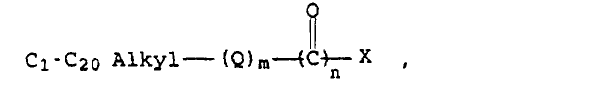 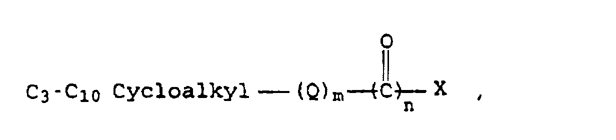 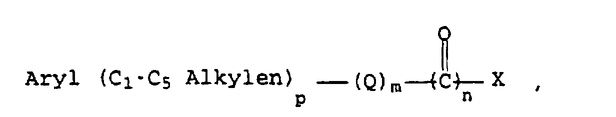 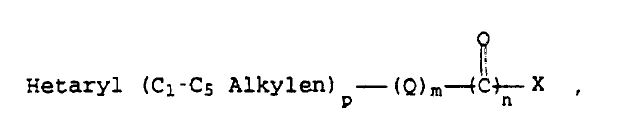 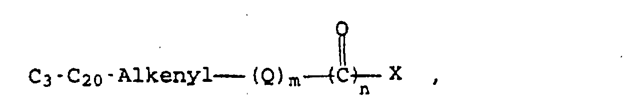 (C₁-C₄ Alykl)₃SiO, Diaryl(C₁-C₅-Alkyl)SiO, Aryl(C₁-C₅-Dialkyl)SiO oder R⁷ und m = 0,1; n = 0,1; p = 0,1 oder (Aryl)₃CO,
R⁷ eine Verbindung der Formel IV bedeutet 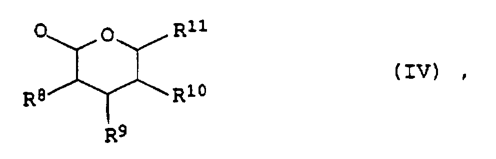 wobei die Substituenten R⁸, R⁹, R¹⁰, R¹¹ folgende Bedeutung haben:
R⁸, R⁹, R¹⁰ unabhängig voneinander, die für die Reste R², R³, R⁴ beschriebene Bedeutung haben und gleich oder verschieden von den Resten R², R³, R⁴ sein können,
R¹¹ CHR⁵R⁶, R⁵
oder zwei benachbarte Reste R⁸, R⁹, R¹⁰, R⁶ unabhängig voneinander ein substituiertes oder unsubstituiertes Arylalkylidenacetal oder ein Alkylidenacetal bilden können,
Q O, NH
X O, NR¹²
Y O, S
R¹² Wasserstoff, substituiertes oder unsubstituiertes C₁-C₁₀-Alkyl, C₃-C₈-Cycloalkyl, Aryl, Hetaryl, Arylalkyl
dadurch gekennzeichnet, daß man Verbindungen der Formel II 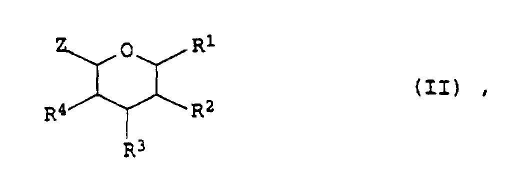 in der Z ausgewählt ist aus der Gruppe
N³, Halogen, 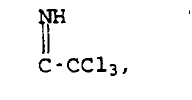 OCH₂CH₂CH₂CH=CH₂, OPO₂H₂, S-Aryl, S-Alkyl
in Gegenwart eines Promotors an eine funktionalisierte feste Phase der Formel III 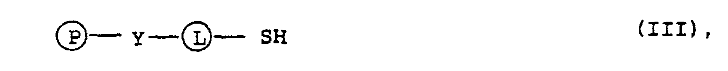 koppelt.

2. Verfahren zur Herstellung von Verbindungen der Formel I nach Anspruch 1, dadurch gekennzeichnet, daß man eine Verbindung der Formel Ia 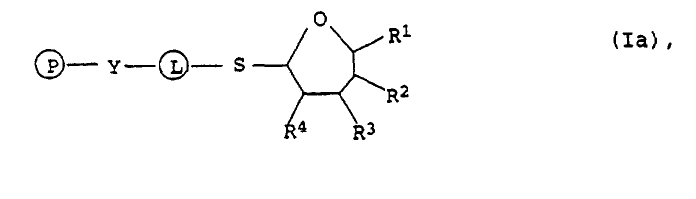 deren Reste R¹ bis R¹² und deren Variablen (P), (L), Q, X, Y, n, m und p die in Anspruch 1 für Formel I genannte Bedeutung haben, mit der Maßgabe, daß mindestens einer der Reste R², R³. R⁴ oder R⁶ XH bedeutet, mit einer Ve=bindung der Formel II in Gegenwart eines Promotors umsetzt.

3. Verfahren nach Anspruch 2, dadurch gekennzeichnet, daß man Verbindungen der Formel Ia mit mehr, gleich oder weniger als einem molaren Äquivalent von Verbindungen der Formel II, bezogen auf die XH Gruppen von Formel Ia, umsetzt.

4. Verfahren zur Herstellung von Verbindungen der Formel I nach den Ansprüchen 1 bis 3, dadurch gekennzeichnet, daß man Verbindungen der Formel Ia mit einem oder mehreren Acylierungs-, Carbamoylierungs- oder Alkylierungsreagentien umsetzt.

5. Verbindungen der Formel I 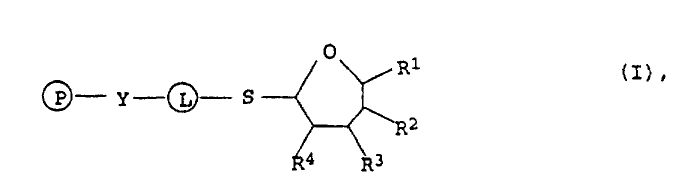 in der die Variable und Substituenten folgende Bedeutung haben:
(P) eine feste Phase
(L) ein aliphatischer Linker mit 2 bis 12 Kohlenstoffatomen,
R¹ CHR⁵R⁶, R⁵
R², R³, R⁴ unabhängig voneinander Wasserstoff, XH, substituiertes oder unsubstituiertes 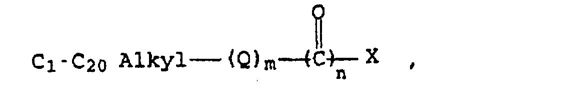 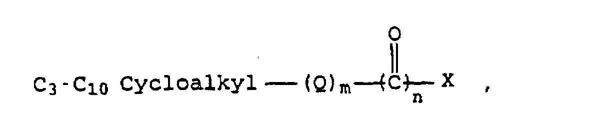 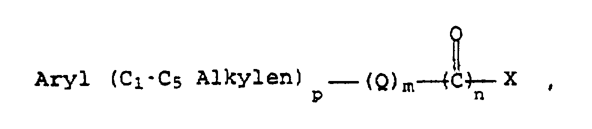 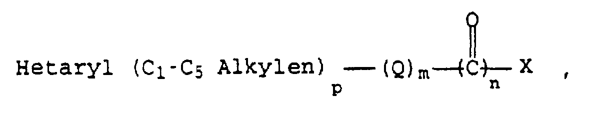 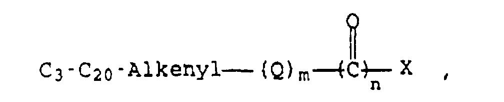 (C₁-C₄-Alykl)₃SiO, Diaryl(C₁-C₅-Alkyl)SiO, Aryl(C₁-C₅-Dialkyl)SiO oder R⁷ und m = 0,1; n = 0,1; p = 0,1
oder zwei benachbarte Reste R², R³, R⁴, R⁶ unabhängig voneinander ein substituiertes oder unsubstituiertes Arylalkylidenacetal oder ein Alkylidenacetal bilden,
R⁵ Wasserstoff, substituiertes oder unsubstituiertes C₁-C₂₀-Alkyl, C₃-C₂₀-Alkenyl, Arylalkylen oder Aryl,
R⁶ Wasserstoff, XH, substituiertes oder unsubstituiertes 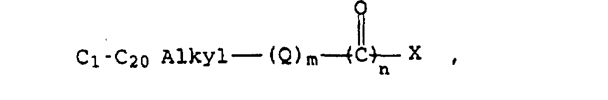 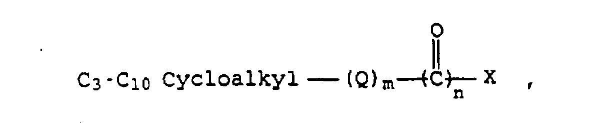 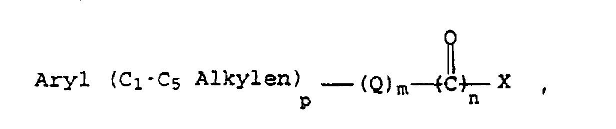 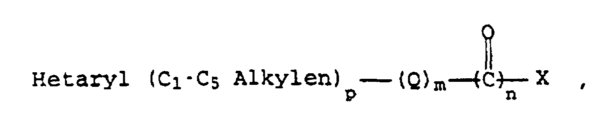 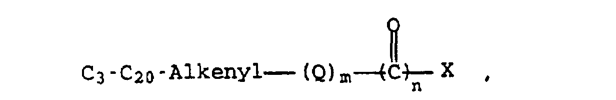 (C₁-C₄-Alykl)₃SiO, Diaryl(C₁-C₅-Alkyl)SiO, Aryl(C₁-C₅-Dialkyl)SiO oder R⁷ und m = 0,1; n = 0,1; p = 0,1 oder (Aryl)₃CO,
R⁷ eine Verbindung der Formel IV bedeutet 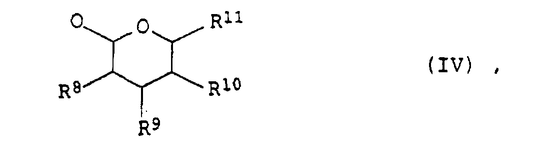 wobei die Substituenten R⁸, R⁹, R¹⁰, R¹¹ folgende Bedeutung haben:
R⁸, R⁹, R¹⁰ unabhängig voneinander, die für die Reste R², R³, R⁴ beschriebene Bedeutung haben und gleich oder verschieden von den Resten R², R³, R⁴ sein können,
R¹¹ CHR⁵R⁶, R⁵
oder zwei benachbarte Reste R⁸, R⁹, R¹⁰, R⁶ unabhängig voneinander ein substituiertes oder unsubstituiertes Arylalkylidenacetal oder ein Alkylidenacetal bilden können,
Q O, NH
X O, NR¹²
Y O, S
R¹² Wasserstoff, substituiertes oder unsubstituiertes C₁-C₁₀-Alkyl, C₃-C₈-Cycloalkyl, Aryl, Hetaryl, Arylalkyl.

6. Funktionalisierte feste Phase der Formel III 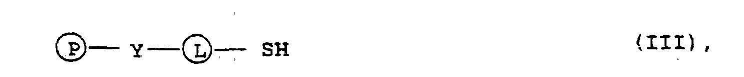 in der
(P) eine feste Phase
(L) einen aliphatischen Linker mit 2 bis 12 Kohlenstoffatomen,
Y O, S
bedeutet.

7. Verbindungen der Formel III nach Anspruch 6, wobei die feste Phase (P) Keramik, Glas, Latex, funktionalisierte quervernetzte Polystyrole, Polyacrylamide, Silicagele oder Harze bedeutet.

8. Verwendung eines Verfahrens gemäß den Ansprüchen 1 bis 4 zur Herstellung von Substanzbibliotheken.

9. Verfahren zur Herstellung von Substanzbibliotheken, die eine Vielzahl von Verbindungen der allgemeinen Formel V, 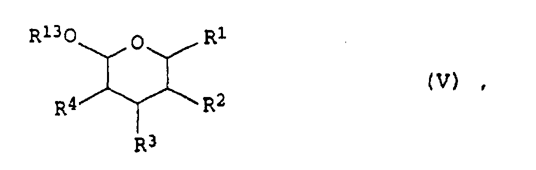 enthalten,
in der die Reste R¹ bis R⁴, die unter Anspruch 5 genannte Bedeutung haben, und R¹³ substituiertes oder unsubstituiertes C₃-C₂₀-Alkenyl, C₁-C₂₀-Alkyl, C₁-C₅-Alkylhetaryl, C₃-C₁₀-Cycloalkyl oder ein- bis viermal verbrücktes substituiertes oder unsubstitutiertes C₅-C₂₅-Cycloalkyl, das mit mindestens einem Substituenten ausgewählt aus der Gruppe C₁-C₁₀-Alkyl, C₃-C₁₀-Alkenyl, CO, COO-(C₁-C₁₀-Alkyl), OH, O-(C₁-C₁₀-Alkyl), O-Alkylhetaryl oder O-Alkylaryl substituiert sein kann oder substituierte oder unsubstituierte Mono-, Di- oder Oligosaccharide oder Hydroxycarbonsäuren, bedeutet, dadurch gekennzeichnet, daß man Verbindungen der Formel I nach Anspruch 5 in Gegenwart mindestens eines thiophilen Reagenz und mindestens eines Alkohols (= R¹³OH) von der funktionalisierten festen Phase abspaltet.

10. verfahren zur Herstellung von Substanzbibliotheken, die eine Vielzahl von Verbindungen der allgemeinen Formel V 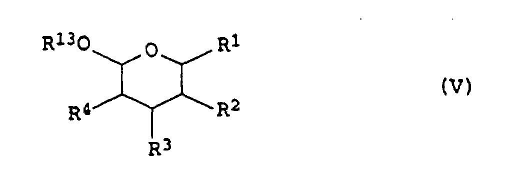 enthalten,
in der die Reste R¹ bis R⁴, die unter Anspruch 5 genannte Bedeutung haben, und R¹³ Wasserstoff bedeutet, dadurch gekennzeichnet, daß man Verbindungen der Formel I nach Anspruch 5 in Gegenwart von Bromonium-Ionen von der funktionalisierten festen Phase abspaltet.

11. Verwendung der nach den Ansprüchen 8 bis 10 erhaltenen Substanzbibliotheken im Massenscreening.

12. Verwendung von Verbindungen der Formel III gemäß Anspruch 6, als funktionalisierte feste Phase für organische Synthesen, für chromatographische Verfahren, zur Immobilisierung von Wirkstoffen oder Antigenen.

## Claims

1. A process for preparing carbohydrate derivatives of the formula I 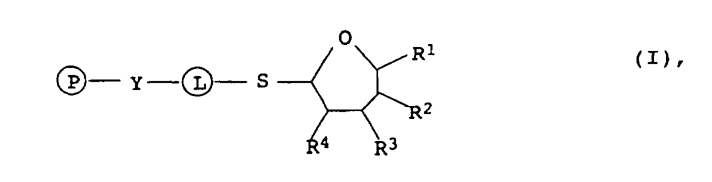 in which the variable and substituents have the following meanings:
(P) a solid phase
(L) an aliphatic linker having 2 to 12 carbon atoms,
R¹ CHR⁵R⁶, R⁵
R², R³, R⁴ independently of one another hydrogen, XH, substituted or unsubstituted 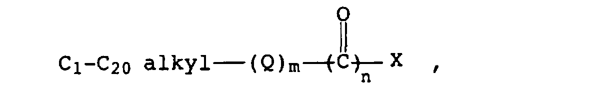 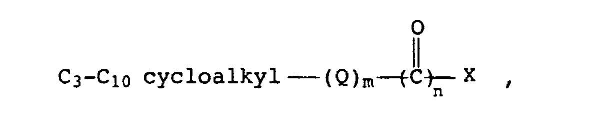 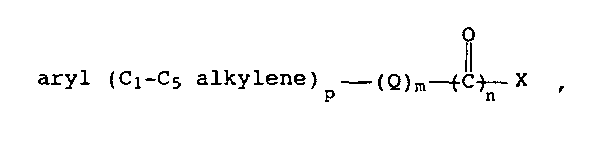 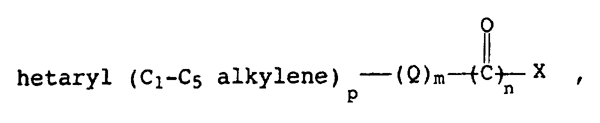 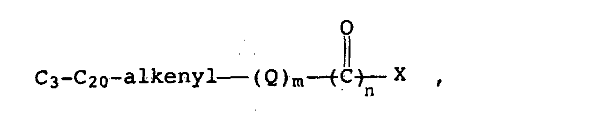 (C₁-C₄-alkyl)₃SiO, diaryl(C₁-C₅-alkyl)SiO, aryl(C₁-C₅-dialkyl)SiO or R⁷ and m = 0, 1; n = 0, 1; p = 0, 1
or two adjacent radicals R², R³, R⁴, R⁶ independently of one another form a substituted or unsubstituted arylalkylidene acetal or an alkylidene acetal,
R⁵ hydrogen, substituted or unsubstituted C₁-C₂₀-alkyl, C₃-C₂₀ alkenyl, arylalkyl or aryl,
R⁶ hydrogen, XH, substituted or unsubstituted 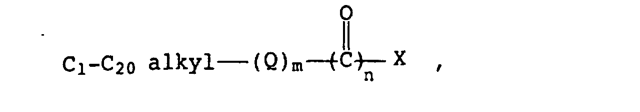 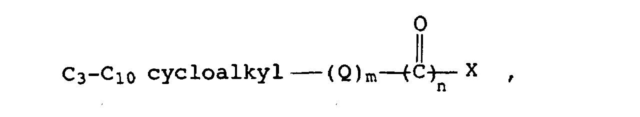 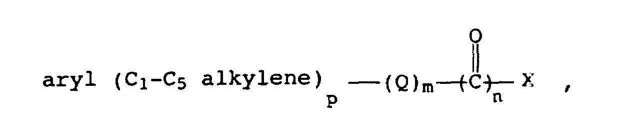 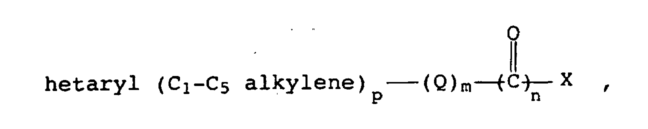 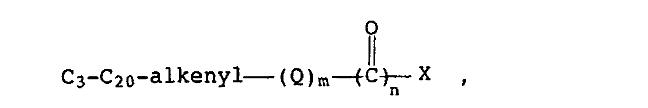 (C₁-C₄ alkyl)₃SiO, diaryl(C₁-C₅-alkyl)SiO, aryl(C₁-C₅-dialkyl)SiO or R⁷ and m = 0, 1; n = 0, 1; p = 0, 1 or (aryl)₃CO,
R⁷ is a group of the formula IV 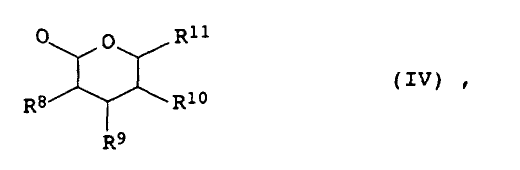 where the substituents R⁸, R⁹, R¹⁰, R¹¹ have the following meanings:
R⁸, R⁹, R¹⁰ independently of one another have the meanings described for the radicals R², R³, R⁴ and may be identical to or different from the radicals R², R³, R⁴,
R¹¹ CHR⁵R⁶, R⁵
or two adjacent radicals R⁸, R⁹, R¹⁰, R⁶ independently of one another may form a substituted or unsubstituted arylalkylidene acetal or an alkylidene acetal,
Q O, NH
X O, NR¹²
Y O, S
R¹² hydrogen, substituted or unsubstituted C₁-C₁₀-alkyl, C₃-C₈-cycloalkyl, aryl, hetaryl, arylalkyl
which comprises coupling compounds of the formula II 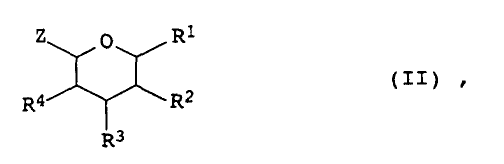 where Z is selected from the group
N₃, halogen, 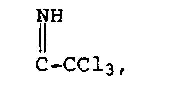 OCH₂CH₂CH₂CH=CH₂, OPO₂H₂, S-aryl, S-alkyl
in the presence of a promotor to a functionalized solid phase of the formula III

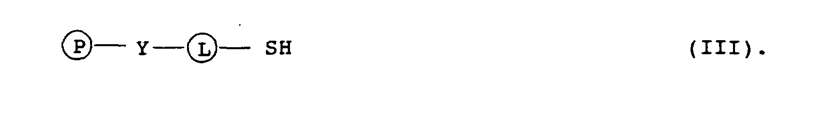

2. A process for preparing compounds of the formula I as claimed in claim 1, wherein a compound of the formula Ia 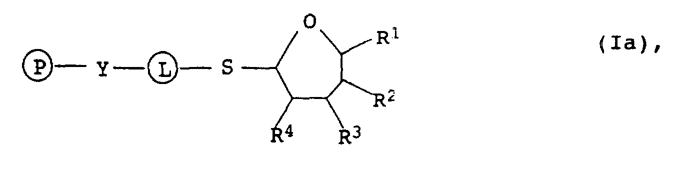 whose radicals R¹ to R¹² and whose variables (P), (L), Q, X, Y, n, m and p have the meanings stated for formula I in claim 1, with the proviso that at least one of the radicals R², R³, R⁴ or R⁶ is XH, is reacted with a compound of the formula II in the presence of a promoter.

3. A process as claimed in claim 2, wherein compounds of the formula Ia are reacted with more than, exactly or less than one molar equivalent of the compounds of the formula II based on the XH groups in formula Ia.

4. A process for preparing compounds of the formula I as claimed in claims 1 to 3, wherein compounds of the formula Ia are reacted with one or more acylating, carbamoylating or alkylating reagents.

5. A compound of the formula I 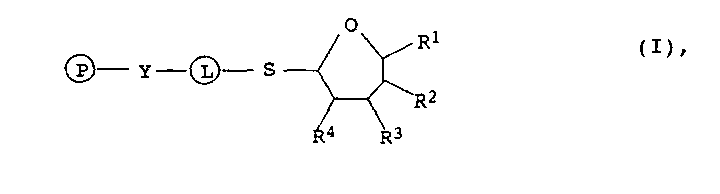 in which the variables and substituents have the following meanings:
(P) a solid phase
(L) an aliphatic linker having 2 to 12 carbon atoms,
R¹ CHR⁵R⁶, R⁵
R², R³, R⁴ independently of one another hydrogen, XH, substituted or unsubstituted 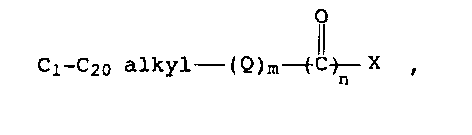 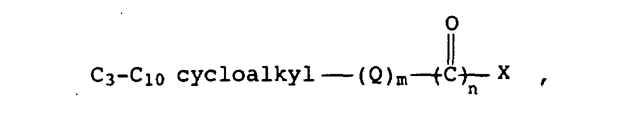 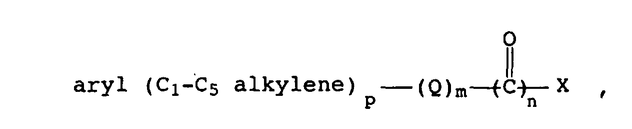 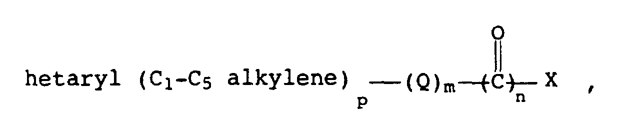 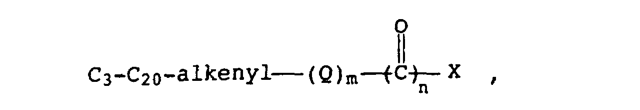 (C₁-C₄-alkyl)₃SiO, diaryl(C₁-C₅-alkyl)SiO, aryl(C₁-C₅-dialkyl)SiO or R⁷ and m = 0, 1; n = 0, 1; p = 0, 1
or two adjacent radicals R², R³, R⁴, R⁶ independently of one another form a substituted or unsubstituted arylalkylidene acetal or an alkylidene acetal,
R⁵ hydrogen, substituted or unsubstituted C₁-C₂₀-alkyl, C₃-C₂₀-alkenyl, arylalkyl or aryl,
R⁶ hydrogen, XH, substituted or unsubstituted 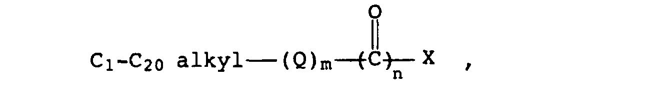 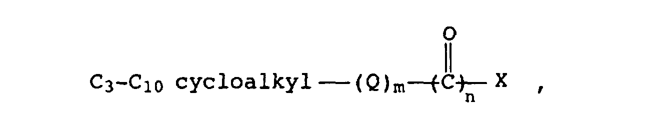 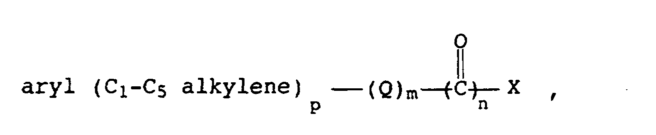 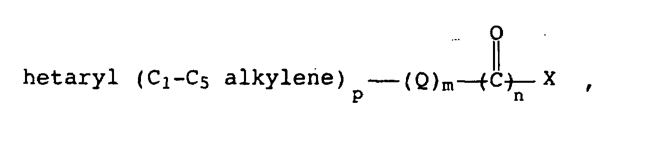 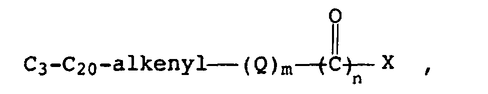 (C₁-C₄-alkyl)₃SiO, diaryl(C₁-C₅-alkyl)SiO, Aryl(C₁-C₅-dialkyl)SiO or R⁷ and m = 0, 1; n = 0, 1; p = 0, 1 or (aryl)₃CO,
R⁷ is a group of the formula IV 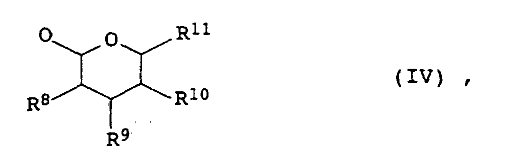 where the substituents R⁸, R⁹, R¹⁰, R¹¹ have the following meanings:
R⁸, R⁹, R¹⁰ independently of one another have the meanings described for the radicals R², R³, R⁴ and may be identical to or different from the radicals R², R³, R⁴,
R¹¹ CHR⁵R⁶, R⁵
or two adjacent radicals R⁸, R⁹, R¹⁰, R⁶ independently of one another may form a substituted or unsubstituted arylalkylidene acetal or an alkylidene acetal,
Q O, NH
X O, NR¹²
Y O, S
R¹² hydrogen, substituted or unsubstituted C₁-C₁₀-alkyl, C₃-C₈-cycloalkyl, aryl, hetaryl, arylalkyl.

6. A functionalized solid phase of the formula III 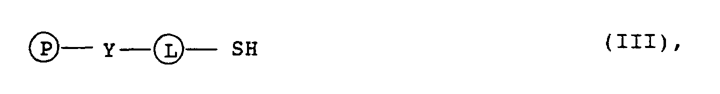 where
(P) is a solid phase
(L) is an aliphatic linker having 2 to 12 carbon atoms,
Y O, S.

7. A compound of the formula III as claimed in claim 6, wherein the solid phase (P) comprises ceramics, glass, latex, functionalized crosslinked polystyrenes, polyacrylamides, silica gels or resins.

8. The use of a process as claimed in any of claims 1 to 4 for preparing substance libraries.

9. A process for preparing substance libraries which comprise a large number of compounds of the general formula V, 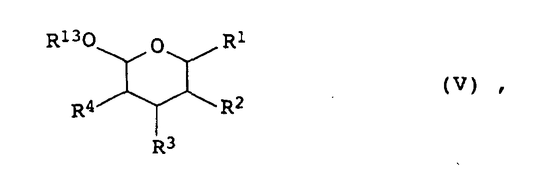 where the radicals R¹ to R⁴ have the meanings stated in claim 5, and R¹³ is substituted or unsubstituted C₃-C₂₀-alkenyl, C₁-C₂₀-alkyl, C₁-C₅-alkylhetaryl, C₃-C₁₀-cycloalkyl or substituted or unsubstituted C₅-C₂₅-cycloalkyl which is bridged once to four times and can be substituted by at least one substituent selected from the group of C₁-C₁₀-alkyl, C₃-C₁₀-alkenyl, CO, COO-(C₁-C₁₀-alkyl), OH, O-(C₁-C₁₀-alkyl), O-alkylhetaryl or O-alkylaryl, or substituted or unsubstituted mono-, di- or oligosaccharides or hydroxy carboxylic acids, which comprises eliminating compounds of the formula I as claimed in claim 5 from the functionalized solid phase in the presence of at least one thiophilic reagent and of at least one alcohol (= R¹³OH).

10. A process for preparing substance libraries which comprise a large number of compounds of the general formula V 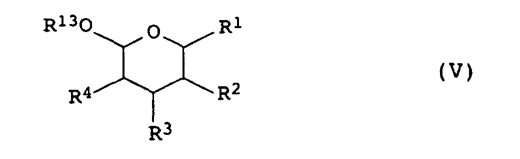 where the radicals R¹ to R⁴ have the meanings stated in claim 5, and R¹³ is hydrogen, which comprises eliminating compounds of the formula I as claimed in claim 5 from the functionalized solid phase in the presence of bromonium ions.

11. The use of the substance libraries obtained as claimed in any of claims 8 to 10 in mass screening.

12. The use of compounds of the formula III as claimed in claim 6 as functionalized solid phase or organic syntheses, for chromatographic methods or for immobilizing active substances or antigens.

## Revendications

1. Procédé pour la préparation de dérivés d'hydrates de carbone de formule I, 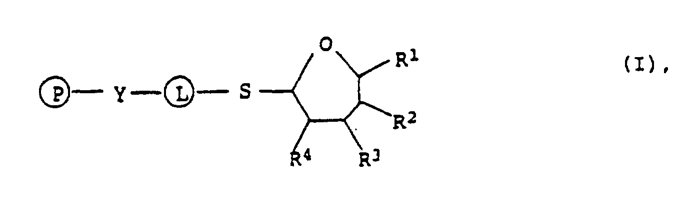 dans laquelle les variables et substituants ont la signification suivante:
(P) une phase solide
(L) un lieur aliphatique ayant 2 à 12 atomes de carbone,
R¹ chR⁵R⁶, R⁵
R², R³, R⁴ indépendamment l'un de l'autre hydrogène, XH, un groupe substitué ou non-substitué 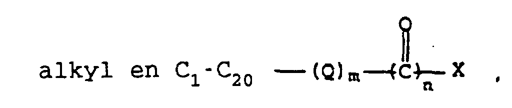 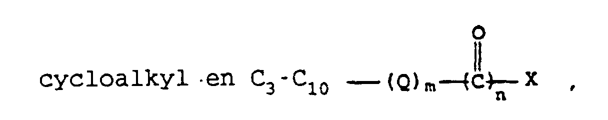 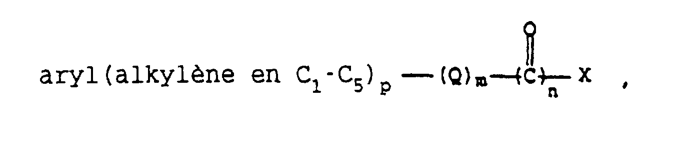 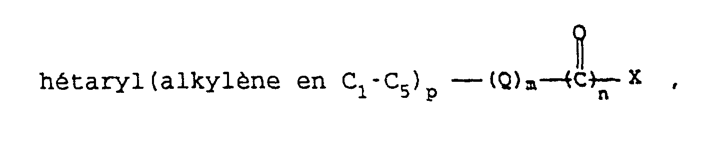 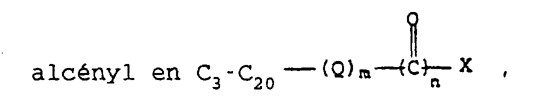 (alkyl en C₁-C₄)₃SiO, diaryl(alkyl en C₁-C₅)-SiO, aryl(dialkyl en C₁-C₅)SiO ou R⁷ et m = 0, 1; n = 0, 1; p = 0, 1
ou deux restes voisins R², R³, R⁴, R⁶ forment, indépendamment l'un de l'autre, un arylalkylidèneacétal ou un alkylidèneacétal substitué ou non-substitué,
R⁵ hydrogène, alkyle en C₁-C₂₀, alcényle en C₃-C₂₀, arylalkylidène ou aryle, substitué ou non-substitué,
R6 hydrogène, XH, un groupe substitué ou non-substitué 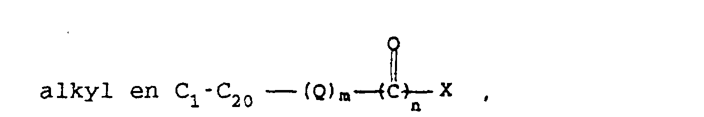 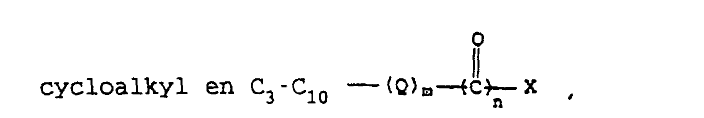 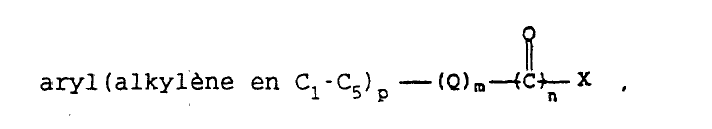 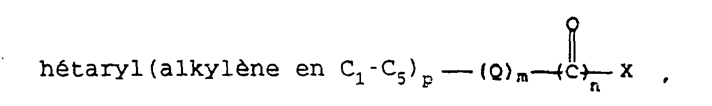 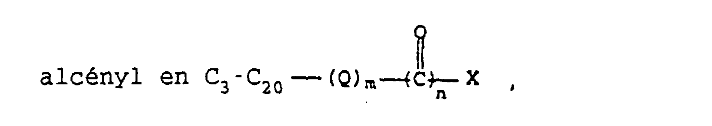 (alkyl en C₁-C₄)₃SiO, diaryl(alkyl en C₁-C₅)-SiO, aryl(dialkyl en C₁-C₅)SiO ou R⁷ et m = 0, 1; n = 0, 1; p = 0, 1 ou (aryl)₃CO,
R⁷ représente un composé de formule 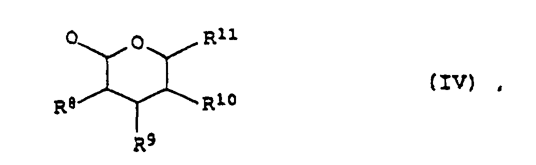 où les substituants R⁸, R⁹, R¹⁰, R¹¹ ont la signification suivante:
R⁸, R⁹, R¹⁰, indépendamment l'un de l'autre, ont la signification indiquée pour les restes R², R³, R⁴ et peuvent être identiques aux ou différents des restes R², R³, R⁴,
R¹¹ CHR⁵R⁶, R⁵
ou deux restes voisins R⁸, R⁹, R¹⁰, R⁶, indépendamment l'un de l'autre, peuvent former un arylalkylidèneacétal ou un alkylidèneacétal, substitué ou non-substitué,
Q O, NH
x O, NR¹²
Y O, S
R¹² hydrogène, ou un groupe substitué ou non-substitué alkyle en C₁-C₁₀, cycloalkyle en C₃-C₈, aryle hétaryle, arylalkyle
caractérisé par le fait qu'on fixe des composés de formule II 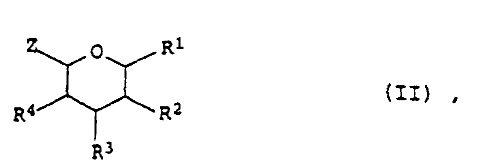 dans laquelle Z est choisi dans le groupe de N³, halogéno 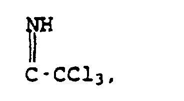 OCHCH₂CH₂CH=CH₂, OPO₂H₂, S-aryle, S-alkyle
en présence d'un promoteur sur une phase solide fonctionnalisée de formule III

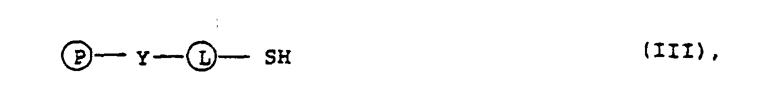

2. Procédé pour la préparation de composés de formule I selon la revendication 1, caractérisé par le fait qu'on fait réagir un composé de formule Ia 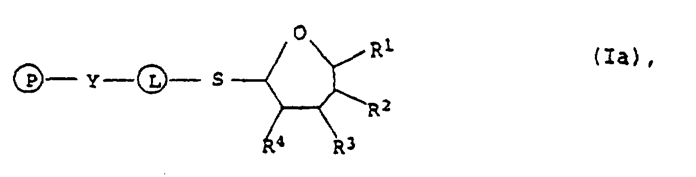 dont les restes R¹ à R¹² et les variables (P) , (L) , Q, X, Y, n, m et p ont la signification indiquée dans la revendication 1 pour la formule I, avec pour condition qu'au moins l'un des restes R², R³, R⁴ ou R⁶ représente XH, avec un composé de formule II en présence d'un promoteur.

3. Procédé selon la revendication 2, caractérisé par le fait qu'on fait réagir des composés de formule Ia avec une proportion supérieure, égale ou inférieure à un équivalent molaire de composés de formule II, par rapport aux groupes XH de la formule Ia.

4. Procédé pour la préparation de composés de formule I selon les revendications 1 à 3, caractérisé par le fait qu'on fait réagir des composés de formule Ia avec un ou plusieurs réactifs d'acylation, de carbamoylation ou d'alkylation.

5. Composés de formule I 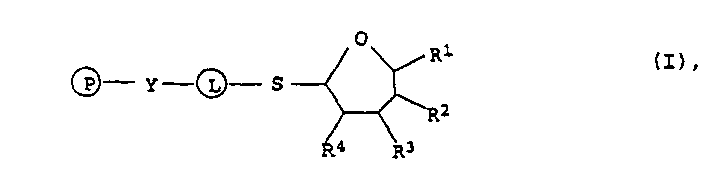 dans laquelle les variables et substituants ont la signification suivante:
(P) une phase solide
(L) un lieur aliphatique ayant 2 à 12 atomes de carbone,
R¹ CHR⁵R⁶, R⁵
R², R³, R⁴, indépendamment l'un de l'autre, hydrogène, XH, un groupe substitué ou non-substitué 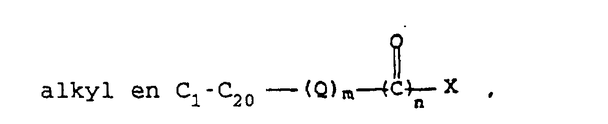 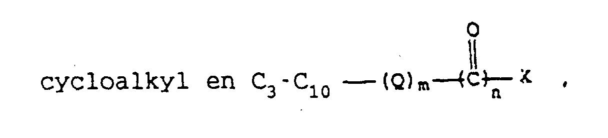 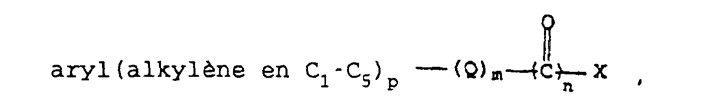 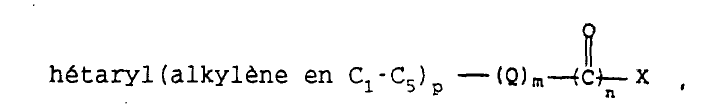 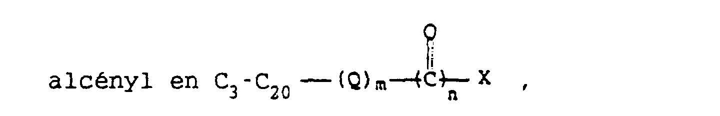 (alkyl en C₁-C₄)₃SiO, diaryl(alkyl en C₁-C₅)-SiO, aryl(dialkyl en C₁-C₅)SiO ou R⁷, et m = 0, 1; n = 0, 1; p = 0, 1
ou deux restes voisins R², R³, R⁴, R⁶ forment, indépendamment l'un de l'autre, un arylalkylidèneacétal ou un alkylidèneacétal substitué ou non-substitué,
R⁵ hydrogène, alkyle en C₁-C₂₀ alcényle en C₃-C₂₀, arylalkylène ou aryle, substitué ou non-substitué,
R⁶ hydrogène, XH, un groupe substitué ou non-substitué 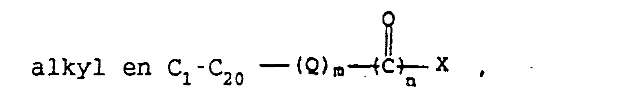 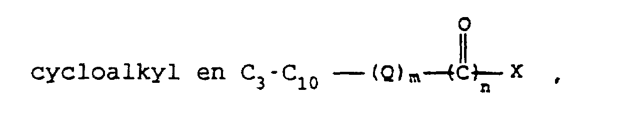 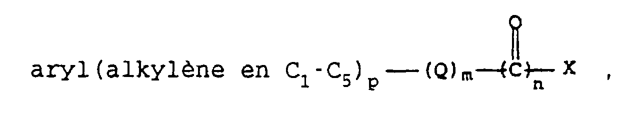 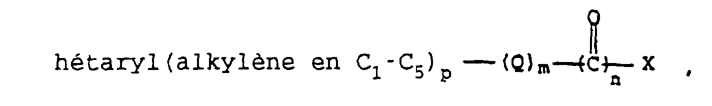 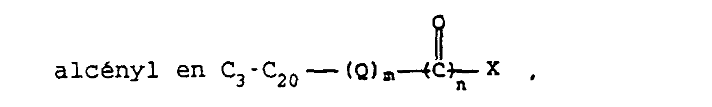 (alkyl en C₁-C₄)₃SiO, diaryl(alkyl en C₁-C₅)SiO, aryl(dialkyl en C₁-C₅)SiO ou R⁷, et m = 0, 1; n = 0, 1; p = 0, 1 ou (aryl)₃CO,
R⁷ désigne un composé de formule IV 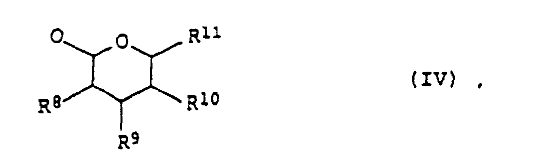 où les substituants R⁸, R⁹, R¹⁰, R¹¹ ont la signification suivante:
R⁸, R⁹, R¹⁰, indépendamment l'un de l'autre, ont la signification indiquée pour les restes R², R³, R⁴ et peuvent être identiques aux ou différents des restes R², R³, R⁴,
R¹¹ CHR⁵R⁶, R⁵
ou deux restes voisins R⁸, R⁹, R¹⁰, R⁶, indépendamment l'un de l'autre, peuvent former un arylalkylidèneacétal substitué ou non-substitué ou un alkylidèneacétal,
Q O, NH
X O, NR¹²
Y O, S
R¹² hydrogène, ou un groupe alkyle en C₁-C₁₀. cycloalkyle en C₃-C₈, aryle, hétaryle, arylalkyle substitué ou non-substitué.

6. Phase solide fonctionnalisée de formule III 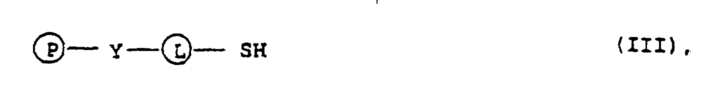 dans laquelle
(P) représente une phase solide
(L) désigne un lieur aliphatique ayant 2 à 12 atomes de carbone,
Y représente O, S.

7. Composés de formule III selon la revendication 6, dans lesquels la phase solide (P) représente de la céramique, du verre, du latex, des polystyrènes réticulés fonctionnalisés, des polyacrylamides, des gels de silice ou des résines.

8. Utilisation d'un procédé selon les revendications 1 à 4 pour la préparation de bibliothèques de substances.

9. Procédé pour la préparation de bibliothèques de substances, qui contiennent une pluralité de composés de formule générale v, 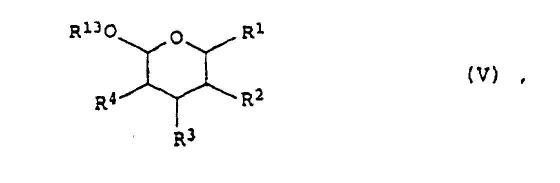 dans laquelle les restes R¹ à R⁴ ont la signification indiquée dans la revendication 5, et R¹³ représente un groupe substitué ou non-substitué alcényle en C₃-C₂₀, alkyle en C₁-C₂₀, alkylhétaryle en C₁-C₅, cycloalkyle en C₃-C₁₀ ou cycloalkyle en C₅-C₂₅ substitué ou non-substitué, ponté une à quatre fois, qui peut être substitué avec au moins un substituant choisi dans le groupe de: alkyle en C₁-C₁₀, alcényle en C₃-C₁₀, COCOO-(alkyle en C₁-C₁₀), OH, O-(alkyle en C₁-C₁₀), O-alkylhétaryle ou O-alkylaryle, ou des mono-, di- ou oligosaccharides ou acides hydroxycarboxyliques, substitués ou non-substitués, caractérisé par le fait qu'on sépare de la phase solide fonctionnalisée des composés de formule I selon la revendication 5 en présence d'au moins un réactif thiophile et d'au moins un alcool (= R¹³OH).

10. Procédé pour la préparation de bibliothèques de substances, qui contiennent une pluralité de composés de formule générale V 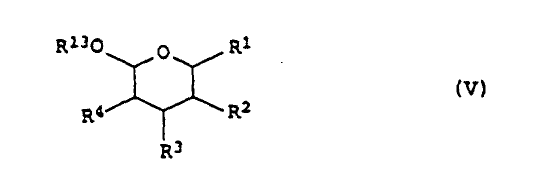 dans laquelle les restes R¹ à R⁴, ont la signification indiquée dans la revendication 5, et R¹³ désigne l'hydrogène, caractérisé par le fait qu'on sépare de la phase solide fonctionnalisée des composés de formule I selon la revendication 5 en présence d'ions bromonium.

11. Utilisation des bibliothèques de substances obtenues selon les revendications 8 à 10 dans le criblage de masses.

12. Utilisation de composés de formule III selon la revendication 6, comme phase solide fonctionnalisée pour synthèses organiques, pour procédés chromatographiques, pour l'immobilisation de substances actives ou d'antigènes.
